(19) Europäisches Patentamt
European Patent Office
Office européen des brevets

(11) **EP 4 600 273 A1**

(12) **EUROPEAN PATENT APPLICATION**
published in accordance with Art. 153(4) EPC

(43) Date of publication:
**13.08.2025 Bulletin 2025/33**

(21) Application number: 23872480.1

(22) Date of filing: 27.09.2023

(51) International Patent Classification (IPC):
C08F 2/10 (2006.01)    A61F 13/53 (2006.01)
C08F 8/00 (2006.01)    C08F 20/00 (2006.01)
C08K 3/30 (2006.01)    C08K 5/00 (2006.01)
C08L 101/14 (2006.01)

(52) Cooperative Patent Classification (CPC):
A61F 13/53; C08F 2/10; C08F 8/00; C08F 20/00;
C08K 3/30; C08K 5/00; C08L 101/14

(86) International application number:
PCT/JP2023/035291

(87) International publication number:
WO 2024/071258 (04.04.2024 Gazette 2024/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 29.09.2022 JP 2022156300

(71) Applicant: Sumitomo Seika Chemicals Co., Ltd.
Kako-gun, Hyogo 675-0145 (JP)

(72) Inventors:
• GOGA, Yuki
Himeji-shi, Hyogo 672-8076 (JP)
• AWAJI, Naoya
Himeji-shi, Hyogo 672-8076 (JP)

(74) Representative: Mewburn Ellis LLP
Aurora Building
Counterslip
Bristol BS1 6BX (GB)

(54) **METHOD FOR PRODUCING WATER ABSORBENT RESIN PARTICLES**

(57)    There is provided a method for producing water-absorbent resin particles, which have good general water absorption performance (for example, physiological saline absorption amount) required for a water-absorbent resin, and also have reduced yellowing under high temperature and high humidity and have high gel stability. A method for producing water-absorbent resin particles comprising, in the following order: step 1 of polymerizing a water-soluble ethylenically unsaturated monomer to obtain hydrous gel particles; step 2 of adding a chelating agent, a sulfite-based compound, and an organic antioxidant to the hydrous gel particles; and step 3 of subjecting the hydrous gel particles to a surface-crosslinking treatment, wherein in step 2, a water content in the hydrous gel particles when adding the sulfite-based compound is 20% by mass or more and 75% by mass or less.

EP 4 600 273 A1

## Description

Technical Field

The present invention relates to a method for producing water-absorbent resin particles, and more particularly relates to a method for producing water-absorbent resin particles that constitute an absorbent material suitably used for hygienic materials, such as disposable diapers, sanitary napkins, and incontinence pads.

Background Art

**In** recent years, water-absorbent resins have been widely used in the field of hygienic materials, such as disposable diapers, sanitary napkins, and incontinence pads.

As such water-absorbent resins, crosslinked products of partially neutralized salt polymers of acrylic acid have been proposed as preferable water-absorbent resins, because they have many advantages. For example, these water-absorbent resins have good water-absorption capacity; acrylic acid used as a raw material is readily industrially available, and thus, they can be produced at low cost with uniform quality; additionally, they are resistant to decomposition or degradation.

Citation List

Patent Literature

Patent Literature 1: JP 2005-29751 A

Summary of Invention

Technical Problem

A gel produced by a water-absorbent resin absorbing a body fluid such as human urine is affected by various components in the body fluid (for example, vitamin C, iron, and the like in urine), which causes a phenomenon in which the gel deteriorates. Therefore, various additives (for example, a human urine stabilizer and the like) are added, or improvements have been made to methods of producing water-absorbent resins.

For example, a sulfite compound as a reducing agent is expected to be effective as a human urine stabilizer and to enhance the gel stability of a water-absorbent resin. However, the addition of a sulfite compound to a water-absorbent resin may cause problems such as deterioration of general water absorption performance (for example, physiological saline absorption amount) of the water-absorbent resin and yellowing of the water-absorbent resin under high temperature and high humidity.

Under such circumstances, it is a main object of the present invention to provide a method for producing water-absorbent resin particles, which have good general water absorption performance (for example, physiological saline absorption amount) required for a water-absorbent resin, and also have reduced yellowing under high temperature and high humidity and have high gel stability.

Solution to Problem

The present inventors have conducted extensive research to solve the aforementioned problem. As a result, the inventors have found that water-absorbent resin particles having good general water absorption performance required for a water-absorbent resin and also having reduced yellowing under high temperature and high humidity and having high gel stability are satisfactorily produced according to a method in which hydrous gel particles obtained by polymerizing a water-soluble ethylenically unsaturated monomer are subjected to a surface-crosslinking treatment to obtain water-absorbent resin particles, which method performs the step of adding a chelating agent, a sulfite-based compound, and an organic antioxidant to the hydrous gel particles before being subjected to the surface-crosslinking treatment, wherein in this step, the water content in the hydrous gel particles when adding the sulfite-based compound falls in a predetermined range. The present invention has been accomplished as a result of further research based on these findings.

In summary, the present invention provides aspects of the invention comprising the following features:

Item 1. A method for producing water-absorbent resin particles comprising, in the following order:

step 1 of polymerizing a water-soluble ethylenically unsaturated monomer to obtain hydrous gel particles;

step 2 of adding a chelating agent, a sulfite-based compound, and an organic antioxidant to the hydrous gel particles; and

step 3 of subjecting the hydrous gel particles to a surface-crosslinking treatment,

wherein in step 2, a water content in the hydrous gel particles when adding the sulfite-based compound is 20% by mass or more and 75% by mass or less.

Item 2. The method according to item 1, wherein step 2 further includes the step of adjusting the water content in the hydrous gel particles.

Item 3. The method according to item 1 or 2, wherein in step 2, the water content in the hydrous gel particles when adding the chelating agent is 20% by mass or more.

Item 4. The method according to any one of items 1 to 3, wherein in step 2, the water content in the hydrous gel particles when adding the organic antioxidant is 20% by mass or more.

Item 5. The method according to any one of items 1 to 4, wherein in step 2, an amount of the sulfite-based compound to be added is 0.001 parts by mass or more and 3.0 parts by mass or less, per 100 parts by mass of the water-soluble ethylenically unsaturated monomer.

Item 6. The method according to any one of items 1 to 5, wherein in step 2, an amount of the chelating agent to be added is 0.001 parts by mass or more and 2.0 parts by mass or less, per 100 parts by mass of the water-soluble ethylenically unsaturated monomer.

Item 7. The method according to any one of items 1 to 6, wherein in step 2, an amount of the organic antioxidant to be added is 0.1 parts by mass or more and 20 parts by mass or less, per 100 parts by mass of the sulfite-based compound.

Item 8. Water-absorbent resin particles,

having a physiological saline absorption amount of 40 to 70 g/g,

having a yellowness index of less than 40 after standing for 14 days in an environment of 70°C and 90% relative humidity, and

having a gel strength of 5500 N/m$^2$ or more after standing for 14 hours in an environment of 37°C and 60% relative humidity.

Item 9. An absorbent material comprising the water-absorbent resin particles according to item 8.

Item 10. An absorbent article comprising the absorbent material according to item 9.

Advantageous Effects of Invention

According to the present invention, it is possible to provide a method for producing water-absorbent resin particles, which have good general water absorption performance (for example, physiological saline absorption amount) required for a water-absorbent resin, and also have reduced yellowing under high temperature and high humidity and have high gel stability. Furthermore, according to the present invention, it is also possible to provide water-absorbent resin particles having a good physiological saline absorption amount, having reduced yellowing under high temperature and high humidity, and having high gel stability; an absorbent material comprising the water-absorbent resin particles; and an absorbent article comprising the absorbent material.

Brief Description of Drawings

Fig. 1 is a schematic diagram of a measurement apparatus for physiological saline absorption amount under a load of 4.14 kPa.

Fig. 2 is a schematic diagram of a measurement apparatus used for measurement of gel strength.

Description of Embodiments

As used herein, the term "comprising" includes "consisting essentially of" and "consisting of". As used herein, the term "(meth)acrylic" refers to "acrylic or methacrylic", and the term "(meth)acrylate" refers to "acrylate or methacrylate". As used herein, the term "water-soluble" refers to having a water solubility of 5% by mass or more at 25°C.

As used herein, values connected with "to" refer to the numerical range including the values before and after "to" as the lower and upper limits. When a plurality of lower limits and a plurality of upper limits are mentioned separately, any lower limit and any upper limit may be selected and connected with "to".

## 1. Method for Producing Water-Absorbent Resin Particles

A method for producing water-absorbent resin particles of the present invention comprises, in the following order: step 1 of polymerizing a water-soluble ethylenically unsaturated monomer to obtain hydrous gel particles; step 2 of adding a chelating agent, a sulfite-based compound, and an organic antioxidant to the hydrous gel particles; and step 3 of subjecting the hydrous gel particles to a surface-crosslinking treatment, wherein in step 2, a water content in the hydrous gel particles when adding the sulfite-based compound is 20% by mass or more and 75% by mass or less. Water-absorbent resin particles produced by the method of the present invention comprising these features have good general water absorption performance (for example, physiological saline absorption amount) required for a water-absorbent resin, and also have reduced yellowing under high temperature and high humidity and have high gel stability. The method for producing water-absorbent resin particles of the present invention will be hereinafter described in detail.

(Step 1)

Step 1 is the step of polymerizing a water-soluble ethylenically unsaturated monomer to obtain hydrous gel particles. A representative polymerization method, such as aqueous solution polymerization, emulsion polymerization, or reversed phase suspension polymerization, is used for polymerizing the water-soluble ethylenically unsaturated monomer. In aqueous solution polymerization, polymerization is performed by heating a water-soluble ethylenically unsaturated monomer solution, optionally with stirring. In reversed phase suspension polymerization, polymerization is performed by heating the water-soluble ethylenically unsaturated monomer with stirring in a hydrocarbon dispersion medium.

Step 1 may further include the step of grinding a hydrous gel obtained by polymerizing the water-soluble ethylenically unsaturated monomer to obtain the hydrous gel particles. When reversed phase suspension polymerization is employed as the polymerization method for the hydrous gel particles, the hydrous gel particles are produced by the polymerization, and thus, the grinding of the hydrous gel is usually unnecessary. As one example of step 1 to obtain the hydrous gel particles, an exemplary method of reversed phase suspension polymerization will be hereinafter described.

A method for obtaining hydrous gel particles by performing reversed phase suspension polymerization of the water-soluble ethylenically unsaturated monomer in a hydrocarbon dispersion medium performs the polymerization in the presence of a radical polymerization initiator. As described below, an internal-crosslinking agent may be optionally added to the water-soluble ethylenically unsaturated monomer to obtain hydrous gel particles having an internal-crosslinked structure.

<Polymerization Step>

[Water-Soluble Ethylenically Unsaturated Monomer]

Examples of the water-soluble ethylenically unsaturated monomer include (meth)acrylic acid ("acryl" and "methacryl" are herein collectively referred to as "(meth)acryl"; the same applies below) and salts thereof; 2-(meth) acrylamido-2-methylpropanesulfonic acid and salts thereof; nonionic monomers, such as (meth)acrylamide, N,N-dimethyl(meth)acrylamide, 2-hydroxyethyl(meth)acrylate, N-methylol(meth)acrylamide, and polyethylene glycol mono(meth)acrylate; and amino group-containing unsaturated monomers, such as N,N-diethylaminoethyl (meth)acrylate, N,N-diethylaminopropyl (meth)acrylate, and diethylaminopropyl (meth)acrylamide, as well as quaternary compounds thereof. Preferred among these water-soluble ethylenically unsaturated monomers are (meth)acrylic acid and salts thereof, (meth)acrylamide, and N,N-dimethyl(meth)acrylamide, and more preferred are (meth)acrylic acid and salts thereof, because they are readily industrially available, for example. These water-soluble ethylenically unsaturated monomers may be used alone or in combinations of two or more.

Among these water-soluble ethylenically unsaturated monomers, acrylic acid and salts thereof are widely used as raw materials of water-absorbent resins. Copolymers of acrylic acid and/or salts thereof with other water-soluble ethylenically unsaturated monomers as mentioned above may also be used. In this case, acrylic acid and/or a salt thereof as a main water-soluble ethylenically unsaturated monomer is preferably used in an amount of 70 to 100 mol% based on the total amount of water-soluble ethylenically unsaturated monomers.

The water-soluble ethylenically unsaturated monomer may be dispersed as an aqueous solution in a hydrocarbon dispersion medium and then subjected to reversed phase suspension polymerization. When the water-soluble ethylenically unsaturated monomer is in the form of an aqueous solution, the dispersion efficiency in the hydrocarbon dispersion medium can be increased. The concentration of the water-soluble ethylenically unsaturated monomer in the aqueous solution is preferably in the range of 20% by mass to not more than the saturated concentration. The concentration of the water-soluble ethylenically unsaturated monomer is more preferably 55% by mass or less, still more preferably 50% by mass or less, and even more preferably 45% by mass or less. On the other hand, the concentration of the water-soluble ethylenically unsaturated monomer is more preferably 25% by mass or more, still more preferably 28% by mass or more, and even more preferably 30% by mass or more.

When the water-soluble ethylenically unsaturated monomer has an acid group such as (meth)acrylic acid or 2-(meth)acrylamido-2-methylpropanesulfonic acid, the acid group may be optionally neutralized with an alkaline neutralizing agent, before the water-soluble ethylenically unsaturated monomer is used. Examples of such alkaline neutralizing agents include alkali metal salts, such as sodium hydroxide, sodium carbonate, sodium hydrogen carbonate, potassium hydroxide, and potassium carbonate; and ammonia. These alkaline neutralizing agents may be used in the form of an aqueous solution to facilitate the neutralization operation. The above-mentioned alkaline neutralizing agents may be used alone or in combinations of two or more.

The neutralization degree of the water-soluble ethylenically unsaturated monomer with an alkaline neutralizing agent, calculated as the neutralization degree of all acid groups in the water-soluble ethylenically unsaturated monomer, is preferably 10 to 100 mol%, more preferably 30 to 90 mol%, still more preferably 40 to 85 mol%, and even more preferably 50 to 80 mol%.

[Radical Polymerization Initiator]

Examples of the radical polymerization initiator to be added in the polymerization step include persulfates, such as potassium persulfate, ammonium persulfate, and sodium persulfate; peroxides, such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds, such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis{2-[1-(2-hydroxyethyl)-2-imidazolin-2-yl]propane} dihydrochloride, 2,2'-azobis{2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid). Preferred among these radical polymerization initiators are potassium persulfate, ammonium persulfate, sodium persulfate, and 2,2'-azobis(2-amidinopropane) dihydrochloride, because they are readily available and easy to handle. These radical polymerization initiators may be used alone or in combinations of two or more. The above-mentioned radical polymerization initiators may also be used in combination with a reducing agent, such as sodium sulfite, sodium hydrogensulfite, ferrous sulfate, or L-ascorbic acid, and used as a redox polymerization initiator.

The amount of the radical polymerization initiator to be used is, for example, 0.00005 to 0.01 mol per mole of the water-soluble ethylenically unsaturated monomer. When the radical polymerization initiator is used in the above-defined range of amounts, the occurrence of an abrupt polymerization reaction can be avoided, and the polymerization reaction can be completed in an appropriate period of time.

[Internal-Crosslinking Agent]

Examples of the internal-crosslinking agent include those that can crosslink the polymer of the water-soluble ethylenically unsaturated monomer to be used, for example; unsaturated polyesters obtained by reacting polyols, such as diols and triols, e.g., (poly)ethylene glycol ["(poly)" means both cases with and without the prefix "poly"; the same applies below], (poly)propylene glycol, 1,4-butanediol, 1,6-hexanediol, trimethylolpropane, and (poly)glycerin, with unsaturated acids, such as (meth)acrylic acid, maleic acid, and fumaric acid; bisacrylamides, such as N,N-methylenebisacrylamide; di or tri(meth)acrylic acid esters obtained by reacting polyepoxides with (meth)acrylic acid; carbamyl di(meth)acrylates obtained by reacting polyisocyanates, such as tolylene diisocyanate and hexamethylene diisocyanate, with hydroxyethyl (meth)acrylate; compounds having two or more polymerizable unsaturated groups, such as allylated starch, allylated cellulose, diallyl phthalate, N,N',N''-triallyl isocyanurate, and divinylbenzene; polyglycidyl compounds, such as diglycidyl compounds, e.g., (poly)ethylene glycol diglycidyl ether, (poly)propylene glycol diglycidyl ether, and (poly)glycerin diglycidyl ether, and triglycidyl compounds; epihalohydrin compounds, such as epichlorohydrin, epibromohydrin, and α-methylepichlorohydrin; compounds having two or more reactive functional groups, such as isocyanate compounds, e.g., 2,4-tolylene diisocyanate and hexamethylene diisocyanate; and oxetane compounds, such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol. Among these internal-crosslinking agents, polyglycidyl compounds are preferably used, diglycidyl ether compounds are more preferably used, and (poly)ethylene glycol diglycidyl ether, (poly)propylene glycol diglycidyl ether, and (poly)glycerin diglycidyl ether are still more preferably used. These internal-crosslinking agents may be used alone or in combinations of two or more.

The amount of the internal-crosslinking agent to be used is preferably 0.000001 to 0.02 mol, more preferably 0.00001 to 0.01 mol, still more preferably 0.00001 to 0.005 mol, and even more preferably 0.00005 to 0.002 mol, per mole of the water-soluble ethylenically unsaturated monomer.

[Hydrocarbon Dispersion Medium]

Examples of the hydrocarbon dispersion medium include $C_{6-8}$ aliphatic hydrocarbons, such as n-hexane, n-heptane, 2-methylhexane, 3-methylhexane, 2,3-dimethylpentane, 3-ethylpentane, and n-octane; alicyclic hydrocarbons, such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons, such as benzene, toluene, and xylene. Among these hydrocarbon dispersion media, n-hexane, n-heptane, and cyclohexane, which are readily industrially available, stable in quality, and inexpensive, are particularly suitably used. These hydrocarbon dispersion media may be used alone or in combinations of two or more. The use of a commercially available mixture of hydrocarbon dispersion media, such as Exxsol Heptane (from Exxon Mobil Corporation; containing 75 to 85% by mass of heptane and its isomeric hydrocarbons), also leads to favorable results.

The amount of the hydrocarbon dispersion medium to be used is preferably 100 to 1500 parts by mass, and more preferably 200 to 1400 parts by mass, per 100 parts by mass of a first-stage water-soluble ethylenically unsaturated monomer, in view of homogeneously dispersing the water-soluble ethylenically unsaturated monomer, and facilitating control of the polymerization temperature. As described below, reversed phase suspension polymerization is performed in one stage (single stage) or two or more multiple stages. The above-mentioned first-stage polymerization refers to the first-stage polymerization reaction in single-stage polymerization or multi-stage polymerization (the same applies below).

[Dispersion Stabilizer]

(Surfactant)

In reversed phase suspension polymerization, a dispersion stabilizer may be used to improve the dispersion stability of the water-soluble ethylenically unsaturated monomer in the hydrocarbon dispersion medium. A surfactant may be used as such a dispersion stabilizer.

Examples of usable surfactants include sucrose fatty acid esters, polyglycerol fatty acid esters, sorbitan fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkyl phenyl ethers, polyoxyethylene castor oil, polyoxyethylene hydrogenated castor oil, alkyl allyl formaldehyde condensate polyoxyethylene ethers, polyoxyethylene-polyoxypropylene block copolymers, polyoxyethylene polyoxypropylene alkyl ethers, polyethylene glycol fatty acid esters, alkyl glucosides, N-alkyl glyconamides, polyoxyethylene fatty acid amides, polyoxyethylene alkylamines, polyoxyethylene alkyl ether phosphates, and polyoxyethylene alkyl allyl ether phosphates. Among these surfactants, sorbitan fatty acid esters, polyglycerol fatty acid esters, and sucrose fatty acid esters are particularly preferably used, in view of dispersion stability of the monomer. These surfactants may be used alone or in combinations of two or more.

The amount of the surfactant to be used is preferably 0.1 to 30 parts by mass, and more preferably 0.3 to 20 parts by mass, per 100 parts by mass of the first-stage water-soluble ethylenically unsaturated monomer.

(Polymeric Dispersion Agent)

A polymeric dispersion agent may be used in combination with the above-described surfactant, as the dispersion stabilizer to be used in reversed phase suspension polymerization.

Examples of the polymeric dispersion agent include maleic anhydride modified polyethylene, maleic anhydride modified polypropylene, maleic anhydride modified ethylene-propylene copolymers, maleic anhydride modified EPDM (ethylene-propylene-diene terpolymers), maleic anhydride modified polybutadiene, maleic anhydride-ethylene copolymers, maleic anhydride-propylene copolymers, maleic anhydride-ethylene-propylene copolymers, maleic anhydride-butadiene copolymers, polyethylene, polypropylene, ethylene-propylene copolymers, oxidized polyethylene, oxidized polypropylene, oxidized ethylene-propylene copolymers, ethylene-acrylic acid copolymers, ethyl cellulose, and ethyl hydroxyethyl cellulose. Among these polymeric dispersion agents, maleic anhydride modified polyethylene, maleic anhydride modified polypropylene, maleic anhydride modified ethylene-propylene copolymers, maleic anhydride-ethylene copolymers, maleic anhydride-propylene copolymers, maleic anhydride-ethylene-propylene copolymers, polyethylene, polypropylene, ethylene-propylene copolymers, oxidized polyethylene, oxidized polypropylene, and oxidized ethylene-propylene copolymers are particularly preferably used, in view of dispersion stability of the monomer. These polymeric dispersion agents may be used alone or in combinations of two or more.

The amount of the polymeric dispersion agent to be used is preferably 0.1 to 30 parts by mass, and more preferably 0.3 to 20 parts by mass, per 100 parts by mass of the first-stage water-soluble ethylenically unsaturated monomer.

[Other Components]

Other components may be optionally added to the aqueous solution containing the water-soluble ethylenically unsaturated monomer, which is then subjected to reversed phase suspension polymerization. Various additives such as thickeners and chain transfer agents can be added as the other components.

By way of example, a thickener may be added to the aqueous solution containing the water-soluble ethylenically unsaturated monomer, which is then subjected to reversed phase suspension polymerization. By thus adding a thickener to adjust the viscosity of the aqueous solution, the median particle size obtained by reversed phase suspension polymerization can be controlled.

Examples of usable thickeners include hydroxyethylcellulose, hydroxypropylcellulose, methylcellulose, carboxymethylcellulose, polyacrylic acid, (partially) neutralized polyacrylic acid, polyethylene glycol, polyacrylamide, polyethyleneimine, dextrin, sodium alginate, polyvinyl alcohol, polyvinylpyrrolidone, and polyethylene oxide. Assuming that the stirring rate during the polymerization is the same, the higher the viscosity of the water-soluble ethylenically unsaturated monomer solution, the larger the primary particles and/or secondary particles of the resulting particles tend to be.

[Reversed Phase Suspension Polymerization]

To perform reversed phase suspension polymerization, for example, an aqueous monomer solution containing the water-soluble ethylenically unsaturated monomer is dispersed in a hydrocarbon dispersion medium, in the presence of a dispersion stabilizer. Here, as long as the dispersion stabilizer (a surfactant and a polymeric dispersion agent) is added before the beginning of the polymerization reaction, it may be added either before or after the addition of the aqueous monomer solution.

In particular, in view of readily reducing the amount of the hydrocarbon dispersion medium remaining in the resulting water-absorbent resin particles, it is preferred to disperse the aqueous monomer solution in the hydrocarbon dispersion medium in which a polymeric dispersion agent is dispersed and then disperse a surfactant therein, followed by polymerization.

Reversed phase suspension polymerization as described above can be performed in one stage or two or more multiple stages. In view of improving productivity, reversed phase suspension polymerization is preferably performed in two or three stages.

Reversed phase suspension polymerization in two or more multiple stages may be performed as follows: the first stage of reversed phase suspension polymerization is performed; subsequently, the water-soluble ethylenically unsaturated monomer is added to and mixed with the reaction mixture obtained by the first-stage polymerization reaction, and the second and subsequent stages of reversed phase suspension polymerization are performed in the same manner as in the first stage. In each of the second and subsequent stages of reversed phase suspension polymerization, it is preferred to add, in addition to the water-soluble ethylenically unsaturated monomer, a radical polymerization initiator within the above-described range of molar ratios of each component relative to the water-soluble ethylenically unsaturated monomer, based on the amount of the water-soluble ethylenically unsaturated monomer added in each of the second and subsequent stages of reversed phase suspension polymerization, and then perform reversed phase suspension polymerization. In the second and subsequent stages of polymerization, an internal-crosslinking agent may also be optionally added to the water-soluble ethylenically unsaturated monomer.

The reaction temperature during the polymerization reaction is preferably 20 to 110°C, and more preferably 40 to 90°C, in view of allowing the polymerization to proceed quickly to reduce the polymerization time for improved economic efficiency, and readily removing the heat of polymerization to perform the reaction smoothly.

(Step 2)

Step 2 is the step of adding a chelating agent, a sulfite-based compound, and an organic antioxidant to the hydrous gel particles obtained in step 1. In the present invention, in step 2, the water content in the hydrous gel particles when adding the sulfite-based compound is 20% by mass or more and 75% by mass or less.

In view of more satisfactorily achieving the effects of the present invention, the water content in the hydrous gel particles when adding the sulfite-based compound to the hydrous gel particles is preferably 20% by mass or more, more preferably 30% by mass or more, and still more preferably 40% by mass or more, with preferred ranges including from 20 to 75% by mass and from 30 to 75% by mass.

Moreover, in view of more satisfactorily achieving the effects of the present invention, the water content in the hydrous gel particles when adding the chelating agent to the hydrous gel particles in step 2 is preferably 20% by mass or more, more preferably 30% by mass or more, and still more preferably 40% by mass or more, while the water content is preferably 150% by mass or less, more preferably 100% by mass or less, and still more preferably 75% by mass or less, with preferred ranges including from 20 to 150% by mass, from 20 to 75% by mass, and from 30 to 75% by mass.

Furthermore, in view of more satisfactorily achieving the effects of the present invention, the water content in the hydrous gel particles when adding the organic antioxidant to the hydrous gel particles in step 2 is preferably 20% by mass or more, more preferably 30% by mass or more, and still more preferably 40% by mass or more, while the water content is preferably 150% by mass or less, more preferably 100% by mass or less, and still more preferably 75% by mass or less, with preferred ranges including from 20 to 150% by mass, from 20 to 75% by mass, and from 30 to 75% by mass.

Preferably, step 2 further includes the step of adjusting the water content in the hydrous gel particles obtained in step 1. For example, when the water content in the hydrous gel particles produced in step 1 is less than 20% by mass, water is added to the hydrous gel particles, or when the water content is more than 75% by mass, the hydrous gel particles are heated to evaporate water, such that the water content can be adjusted within the range of 20% by mass or more and 75% by mass or less. For example, when the hydrous gel particles are produced by, for example, reversed phase suspension polymerization, the water content in the resulting hydrous gel particles is usually more than 75% by mass (for example, the water content is often within the range of 100 to 250% by mass). It is thus effective to include the step of adjusting the water content in the hydrous gel particles obtained in step 1.

The order of adding the chelating agent, the sulfite-based compound, and the organic antioxidant to the hydrous gel particles is not specifically limited. For example, the chelating agent, the sulfite-based compound and the organic antioxidant may be added in this order, or at least two of them may be added at the same time.

In view of more satisfactorily achieving the effects of the present invention, in step 2, the amount of the sulfite-based compound to be added per 100 parts by mass of the water-soluble ethylenically unsaturated monomer is preferably 0.001 parts by mass or more, more preferably 0.01 parts by mass or more, and still more preferably 0.05 parts by mass or more, while the amount is preferably 3.0 parts by mass or less, more preferably 1.0 parts by mass or less, still more preferably 0.50 parts by mass or less, and even more preferably 0.25 parts by mass or less, with preferred ranges including from 0.001 to 3.0 parts by mass and from 0.01 to 1.0 parts by mass.

Moreover, in view of more satisfactorily achieving the effects of the present invention, in step 2, the amount of the chelating agent to be added per 100 parts by mass of the water-soluble ethylenically unsaturated monomer is preferably 0.001 parts by mass or more, more preferably 0.002 parts by mass or more, and still more preferably 0.003 parts by mass or more, while the amount is preferably 2.0 parts by mass or less, more preferably 1.0 parts by mass or less, still more preferably 0.50 parts by mass or less, even more preferably 0.10 parts by mass or less, and still more preferably 0.05 parts by mass or less, with preferred ranges including from 0.001 to 2.0 parts by mass and from 0.002 to 1.0 parts by mass.

Furthermore, in view of more satisfactorily achieving the effects of the present invention, in step 2, the amount of the organic antioxidant to be added per 100 parts by mass of the sulfite-based compound is preferably 0.10 parts by mass or more, and more preferably 0.30 parts by mass or more, while the amount is preferably 20 parts by mass or less, and more preferably 10 parts by mass or less, with preferred ranges including from 0.10 to 20 parts by mass and from 0.30 to 10 parts by mass.

In view of more satisfactorily achieving the effects of the present invention, each of the chelating agent, the sulfite-based compound, and the organic antioxidant is preferably added in the form of an aqueous solution to the hydrous gel particles. The concentration of each component in the aqueous solution may be adjusted so as to achieve the above-described amount to be added. For example, the concentration of the aqueous chelating agent solution to be added to the hydrous gel particles is preferably 0.1 to 50% by mass, and more preferably 0.5 to 40% by mass. The concentration of the aqueous sulfite-based compound solution to be added to the hydrous gel particles is preferably 1 to 20% by mass, and more preferably 5 to 20% by mass. The concentration of the aqueous organic antioxidant solution to be added to the hydrous gel particles is preferably 0.01 to 10% by mass, and more preferably 0.1 to 5% by mass.

In view of more satisfactorily achieving the effects of the present invention, preferred examples of the sulfite-based compound include sodium sulfite, potassium sulfite, calcium sulfite, sodium hydrogensulfite, potassium hydrogensulfite, ammonium hydrogensulfite, sodium pyrosulfite, and potassium pyrosulfite. These sulfite-based compounds may be used alone or in combinations of two or more.

In view of more satisfactorily achieving the effects of the present invention, preferred examples of the chelating agent include ethylenediamine-N,N'-disuccinic acid, diethylenetriaminepentaacetic acid, glycol ether diamine tetraacetic acid, ethylenediaminetetramethylene phosphonic acid, and diethylenetriamine pentamethylene phosphonic acid. The chelating agent may be present in the form of a salt. Examples of the salt include a sodium salt and a potassium salt. These chelating agents may be used alone or in combinations of two or more.

In view of more satisfactorily achieving the effects of the present invention, preferred examples of the organic antioxidant include ascorbic acids, erythorbic acids, gallic acids, protocatechuic acids, benzimidazoles, and alkylhydroxyanisoles. These organic antioxidants may be used alone or in combinations of two or more.

(Step 3)

Step 3 is the step of subjecting the hydrous gel particles to which the chelating agent, the sulfite-based compound, and the organic antioxidant have been added in step 2 to surface-crosslinking. The surface-crosslinking causes the surfaces of the hydrous gel particles to be crosslinked (surface-crosslinking reaction). By thus subjecting the hydrous gel particles having an internal-crosslinked structure to the surface-crosslinking reaction, it is possible to obtain water-absorbent resin particles having an increased crosslinking density near the surfaces of the water-absorbent resin particles to exhibit improvement in various kinds of performance, such as physiological saline absorption amount under load.

Examples of the surface-crosslinking agent include compounds with two or more reactive functional groups. Examples include polyols, such as ethylene glycol, propylene glycol, 1,4-butanediol, diethylene glycol, triethylene glycol, trimethylolpropane, glycerin, polyoxyethylene glycol, polyoxypropylene glycol, and polyglycerin; polyglycidyl compounds, such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds, such as epichlorohydrin, epibromohydrin, and $\alpha$-methyl epichlorohydrin; isocyanate compounds, such as 2,4-tolylene diisocyanate and hexamethylene diisocyanate; oxetane compounds, such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol; oxazoline compounds, such as 1,2-ethylenebisoxazoline; carbonate compounds (e.g., alkylene carbonates), such as ethylene carbonate, propylene carbonate, 4,5-dimethyl-1,3-dioxolan-2-one, 4,4-dimethyl-1,3-dioxolan-2-one, 4-ethyl-1,3-dioxolan-2-one, 4-hydroxymethyl-1,3-dioxolan-2-one, 1,3-dioxan-2-one, 4-methyl-1,3-dioxan-2-one, 4,6-dimethyl-1,3-dioxan-2-one, and 1,3-dioxopan-2-one; and hydroxyalkylamide compounds, such as bis[N,N-di($\beta$-hydroxyethyl)]adipamide. Preferred among these surface-crosslinking agents are polyols, such as ethylene glycol, propylene glycol, 1,4-butanediol, diethylene glycol, triethylene glycol, trimethylolpropane, glycerin, polyoxyethylene glycol, polyoxypropylene glycol, and polyglycerin; polyglycidyl compounds, such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; and carbonate compounds (e.g., alkylene carbonates), such as ethylene carbonate, propylene carbonate, 4,5-dimethyl-1,3-dioxolan-2-one, 4,4-dimethyl-1,3-dioxolan-2-one, 4-ethyl-1,3-dioxolan-2-one, 4-hydroxymethyl-1,3-dioxolan-2-one, 1,3-dioxan-2-one, 4-methyl-1,3-dioxan-2-one, 4,6-dimethyl-1,3-dioxan-2-one, and 1,3-dioxolan-2-one. These surface-crosslinking agents may be used alone or in combinations of two or more.

The amount of the surface-crosslinking agent to be used is preferably 0.00001 to 0.01 mol, more preferably 0.00005 to 0.005 mol, and still more preferably 0.0001 to 0.002 mol, per mole of the total amount of the water-soluble ethylenically unsaturated monomer used for polymerization.

The surface-crosslinking agent may be added as is or as an aqueous solution. Optionally, a solution of the surface-crosslinking agent in a hydrophilic organic solvent may be added. Examples of the hydrophilic organic solvent include lower alcohols, such as methyl alcohol, ethyl alcohol, n-propyl alcohol, and isopropyl alcohol; ketones, such as acetone and methyl ethyl ketone; ethers, such as diethyl ether, dioxane, and tetrahydrofuran; amides, such as N,N-dimethylformamide; and sulfoxides, such as dimethylsulfoxide. These hydrophilic organic solvents may be used alone, in combinations of two or more, or as a solvent mixture with water.

The water content in the hydrous gel particles when adding the surface-crosslinking agent to the hydrous gel particles is preferably in the range of 1 to 75% by mass, more preferably in the range of 5 to 60% by mass, still more preferably in the range of 10 to 50% by mass, and even more preferably in the range of 15 to 40% by mass.

The reaction temperature during the surface-crosslinking reaction is preferably 50 to 250°C, more preferably 60 to 180°C, still more preferably 60 to 140°C, and even more preferably 70 to 120°C. The reaction time of the surface-crosslinking reaction is preferably 1 to 300 minutes, and more preferably 5 to 200 minutes.

Step 3 preferably further includes a drying step of drying the water-absorbent resin particles obtained by surface-treating the hydrous gel particles. Specifically, after reversed phase suspension polymerization is performed as described above, the method may include a drying step of adding external energy, such as heat, to remove the water, the hydrocarbon dispersion medium, and the like by distillation. **In** the case of drying the hydrous gel particles after reversed phase suspension polymerization, the system in which the hydrous gel particles are dispersed in the hydrocarbon dispersion medium is heated to distill the water and the hydrocarbon dispersion medium out of the system by azeotropic distillation. Here, if the distilled hydrocarbon dispersion medium only is returned into the system, continuous azeotropic distillation can be performed. This is preferable in view of preventing deterioration of the resin, because the temperature within the system during drying is maintained at a temperature not higher than the azeotropic temperature with the hydrocarbon dispersion medium. Subsequently, the water and the hydrocarbon dispersion medium are distilled off to obtain dried water-absorbent resin particles. By controlling the treatment conditions for the drying step after the polymerization to adjust the amount of water to be removed, various kinds of performance of the resulting water-absorbent resin particles can be controlled.

In the drying step, the drying treatment by distillation may be performed under atmospheric pressure or reduced pressure. The drying treatment may also be performed in a stream of nitrogen or the like, in view of improving the drying efficiency. When the drying treatment is performed under atmospheric pressure, the drying temperature is preferably 70 to 250°C, more preferably 80 to 180°C, still more preferably 80 to 140°C, and even more preferably 90 to 130°C. When the drying treatment is performed under reduced pressure, the drying temperature is preferably 40 to 160°C, and more preferably 50 to 110°C.

The water-absorbent resin particles obtained by the method of the present invention have a loss on drying of preferably 20% by mass or less, more preferably 15% by mass or less, still more preferably 10% by mass or less, with a preferred range including from 1 to 10% by mass.

When the surface-crosslinking step with a surface-crosslinking agent is performed after the polymerization of the monomer by reversed phase suspension polymerization, the drying step by distillation is performed as described above, after the completion of the surface-crosslinking step. Alternatively, the surface-crosslinking step and the drying step may be performed simultaneously.

The water-absorbent resin particles of the present invention may contain additives suitable for their purpose. Examples of such additives include inorganic powders, surfactants, oxidizing agents, radical chain inhibitors, and anti-bacterial agents. For example, when 0.05 to 5 parts by mass of amorphous silica as an inorganic powder is added to 100 parts by mass of the water-absorbent resin particles, the flowability of the water-absorbent resin particles can be further improved. The additives are preferably hydrophilic or water-soluble. The additives are preferably added to the water-absorbent resin particles obtained after the surface-crosslinking step.

In the water-absorbent resin particles of the present invention, the content of the water-absorbent resin (excluding the additives) including water (loss on drying) is preferably 70% by mass or more, more preferably 80% by mass or more, and still more preferably 90% by mass or more.

The water-absorbent resin particles produced by the method of the present invention have good general water absorption performance (for example, physiological saline absorption amount) required for a water-absorbent resin, and also have reduced yellowing under high temperature and high humidity and have high gel stability.

The water-absorbent resin particles of the present invention have, for example, a physiological saline absorption amount of 40 to 70 g/g, a yellowness index of less than 40 after standing for 14 days in an environment of 70°C and 90% relative humidity, and a gel strength of 5500 N/m$^2$ or more after standing for 14 hours in an environment of 37°C and 60% relative humidity. The water-absorbent resin particles of the present invention are satisfactorily produced by the method of the present invention, but are not limited to the water-absorbent resin particles produced by the above-described method, as long as they satisfy the physiological saline absorption amount, the yellowness index after standing for 14 days in an environment of 70°C and 90% relative humidity, and the gel strength after standing for 14 hours in an environment of 37°C and 60% relative humidity as described above.

The physiological saline absorption amount of the water-absorbent resin particles of the present invention is preferably 40 g/g or more, more preferably 45 g/g or more, and still preferably 50 g/g or more. In view of preventing sliminess of the gel after water absorption, the upper limit value is preferably 70 g/g or less, and preferred ranges include from 40 to 70 g/g and from 45 to 70 g/g.

The water-absorbent resin particles of the present invention have a physiological saline retention amount of preferably 25 g/g or more, more preferably 30 g/g or more. On the other hand, the physiological saline retention amount is preferably 60 g/g or less, more preferably 55 g/g or less, and still more preferably 50 g/g or less. Preferred ranges include from 25 to 60 g/g and from 30 to 55 g/g.

The water-absorbent resin particles produced by the method of the present invention have a physiological saline absorption amount under a load of 4.14 kPa of preferably 5 mL/g or more, more preferably 10 mL/g or more. On the other hand, the physiological saline absorption amount under a load of 4.14 kPa is preferably 40 mL/g or less, more preferably 35 mL/g or less, and still more preferably 30 mL/g or less. Preferred ranges include from 5 to 40 mL/g and from 10 to 30 mL/g.

The physiological saline absorption amount, the physiological saline retention amount, and the physiological saline absorption amount under a load of 4.14 kPa of the water-absorbent resin particles are each the value as measured by the method described in the Examples section.

The water-absorbent resin particles of the present invention have an "initial value of yellowness index" of preferably less than 20, more preferably less than 15, still more preferably less than 10, as evaluated by the method described in the Examples section. The lower limit of the yellowness index is, for example, zero.

The water-absorbent resin particles of the present invention have a "yellowness index after standing for 7 days in an environment of 70°C and 90% relative humidity" of preferably less than 40, more preferably less than 38, still more preferably less than 30, as evaluated by the method described in the Examples section. The lower limit of the yellowness index is, for example, 5.

The water-absorbent resin particles of the present invention have a "yellowness index after standing for 14 days in an environment of 70°C and 90% relative humidity" of preferably less than 40, more preferably less than 38, as evaluated by the method described in the Examples section. The lower limit of the yellowness index is, for example, 10.

The water-absorbent resin particles of the present invention have an "initial value of gel strength" of preferably 5500 N/m$^2$ or more, more preferably 6200 N/m$^2$ or more, as evaluated by the method described in the Examples section. The upper limit of the initial value of gel strength is, for example, 20000 N/m$^2$.

The water-absorbent resin particles of the present invention have a "gel strength after standing at 37°C for 14 hours" of preferably 5500 N/m$^2$ or more, more preferably 6200 N/m$^2$ or more, as evaluated by the method described in the Examples section. The upper limit of the gel strength is, for example, 20000 N/m$^2$.

## 2. Absorbent Material and Absorbent Article

The water-absorbent resin particles of the present invention constitute an absorbent material used for hygienic materials, such as sanitary items and disposable diapers, for example, and is suitably used for an absorbent article including the absorbent material.

Herein, the absorbent material obtained using the water-absorbent resin particles of the present invention contains the water-absorbent resin particles of the present invention. The absorbent material may further contain hydrophilic fibers. Examples of the structure of the absorbent material include a sheet-like structure in which the water-absorbent resin particles are fixed on a nonwoven fabric or between a plurality of nonwoven fabrics; a mixed dispersion obtained by mixing the water-absorbent resin particles and hydrophilic fibers to give a homogeneous composition; a sandwich structure in which the water-absorbent resin particles are sandwiched between layered hydrophilic fibers; and a structure in which the water-absorbent resin particles and hydrophilic fibers are wrapped in a tissue. The absorbent material may also contain other components, for example, adhesive binders such as thermal bonding synthetic fibers, hot melt adhesives, and adhesive emulsions, for improving the shape retention properties of the absorbent material.

The content of the water-absorbent resin particles in the absorbent material is preferably 5 to 100% by mass, more preferably 10 to 95% by mass, still more preferably 20 to 90% by mass, and even more preferably 30 to 80% by mass.

Examples of the hydrophilic fibers include cellulose fibers, such as cotton-like pulp made from wood, mechanical pulp, chemical pulp, and semi-chemical pulp; artificial cellulose fibers, such as rayon and acetate; and fibers made of synthetic resins, such as hydrophilized polyamides, polyesters, and polyolefins. The hydrophilic fibers typically have an average fiber length of 0.1 to 10 mm, or have an average fiber length of 0.5 to 5 mm.

It is possible to obtain the absorbent article of the present invention by holding the absorbent material obtained using the water-absorbent resin particles of the present invention between a liquid-permeable sheet (top sheet) that allows a liquid to pass through and a liquid-impermeable sheet (back sheet) that does not allow a liquid to pass through. The liquid-permeable sheet is positioned on the side that is brought into contact with the body, and the liquid-impermeable sheet is positioned opposite to the side that is brought into contact with the body.

Examples of the liquid-permeable sheet include porous synthetic resin sheets and nonwoven fabrics of the types such as air-through type, spunbond type, chemical bond type, and needle punch type, which are made of fibers of polyethylene, polypropylene, polyester, and the like. Examples of the liquid-impermeable sheet include synthetic resin films made of resins such as polyethylene, polypropylene, and polyvinyl chloride.

## Examples

The present invention will be hereinafter described in detail with reference to examples and comparative examples. However, the water-absorbent resin particles of the present invention and the method for producing the water-absorbent resin particles are not limited to the examples.

The water-absorbent resin particles obtained in the production examples below and the water-absorbent resin particles obtained in the examples and comparative examples below were evaluated using the tests as described below. Unless otherwise indicated, the measurement was performed in an environment at a temperature of 25 $\pm$ 2°C and a relative humidity of 50 $\pm$ 10%.

## <Example 1>

[Step of Producing Hydrous Gel Particles (Step 1)]

An 11 cm inner diameter, 2 L volume, cylindrical round-bottomed separable flask was prepared which was equipped with a reflux condenser, a dropping funnel, a nitrogen gas inlet tube, and an agitator with stirring blades having two stages of four inclined paddle blades with a blade diameter of 5 cm. As a hydrocarbon dispersion medium, 293 g of n-heptane was placed in this flask, and 0.736 g of a maleic anhydride-modified ethylene-propylene copolymer (Hi-Wax 1105A from Mitsui Chemicals, Inc.) was added as a polymeric dispersion agent. The mixture was heated with stirring to 80°C to dissolve the dispersion agent and then cooled to 50°C. Separately, in a 300 mL inner volume beaker, 92.0 g (1.03 mol) of an 80.5% by mass aqueous solution of acrylic acid was placed as a water-soluble ethylenically unsaturated monomer, and 147.7 g of a 20.9% by mass aqueous solution of sodium hydroxide was added dropwise, while cooling with ice water, to achieve 75 mol% neutralization. Then, 0.0736 g (0.272 mmol) of potassium persulfate as a water-soluble radical polymerization initiator and 0.010 g (0.057 mmol) of ethylene glycol diglycidyl ether as an internal-crosslinking agent were added and dissolved to prepare a first-stage aqueous solution. Then, the aqueous solution prepared as above was added to the separable flask and stirred for 10 minutes. Then, a surfactant solution prepared in a 20 mL vial by heating and dissolving 0.736 g of sucrose stearate having an HLB of 3 (Ryoto sugar ester S-370 from Mitsubishi-Kagaku Foods Corporation) as a surfactant in 6.62 g of n-heptane was further added. The system was sufficiently purged with nitrogen while stirring with the agitator at a rotation speed of 500 rpm. Then, the flask was immersed in a water bath at 70°C and heated, and polymerization was performed for 60 minutes to give a first-stage polymerization slurry.

Separately, in another 500 mL inner volume beaker, 128.8 g (1.44 mol) of an 80.5% by mass aqueous solution of acrylic acid was placed as a water-soluble ethylenically unsaturated monomer, and 160.0 g of a 27% by mass aqueous solution of sodium hydroxide was added dropwise, while cooling with ice water, to achieve 75 mol% neutralization. Then, 0.103 g (0.381 mmol) of potassium persulfate as a water-soluble radical polymerization initiator and 0.0116 g (0.067 mmol) of ethylene glycol diglycidyl ether as an internal-crosslinking agent were added and dissolved to prepare a second-stage aqueous solution.

The separable flask system was cooled to 27°C while stirring with the agitator at a rotation speed of 1000 rpm. Then, the entire amount of the second-stage aqueous solution was added to the first-stage polymerization slurry, and the system was purged with nitrogen for 30 minutes. Then, the flask was again immersed in a water bath at 70°C and heated, and the polymerization reaction was performed for 60 minutes to give hydrous gel particles.

[Step of Adding Additives to Hydrous Gel Particles (Step 2)]

After the hydrous gel particles were obtained as above, the flask was immersed in an oil bath set at 125°C to remove 175.1 g of water out of the system by azeotropic distillation of the n-heptane and water, while refluxing the n-heptane (first step of adjusting the water content). Then, 4.42 g of a 0.5% by mass aqueous solution of ethylenediamine-N,N'-disuccinic acid-trisodium salt was added with stirring. Here, the water content in the hydrous gel particles when adding the aqueous solution of ethylenediamine-N,N'-disuccinic acid-trisodium salt was 62% by mass. Subsequently, 2.21 g of a 20% by mass aqueous solution of sodium sulfite was added with stirring. Here, the water content in the hydrous gel particles when adding the aqueous solution of sodium sulfite was 64% by mass. Subsequently, 2.21 g of a 0.2% by mass aqueous solution of L(+)-ascorbic acid was added with stirring. Here, the water content in the hydrous gel particles when adding the aqueous solution of L(+)-ascorbic acid was 65% by mass.

[Surface-Crosslinking Step (Step 3)]

Then again, 84.7 g of water was removed out of the system by azeotropic distillation of the n-heptane and water, while refluxing the n-heptane. Then, 4.42 g (0.507 mmol) of a 2% by mass aqueous solution of ethylene glycol diglycidyl ether was added to the flask as a surface-crosslinking agent, and the flask was maintained at 83°C for 2 hours. Then, the n-heptane was evaporated at 125°C to dry the reaction mixture, and the resulting product was subsequently passed through a sieve with a mesh size of 850 μm to give 225.7 g of water-absorbent resin particles.

[Step of Adding Additives to Water-Absorbent Resin Particles]

100 parts by mass of the obtained water-absorbent resin particles were mixed with 0.5 parts by mass of amorphous silica (Tokusil NP-S from Oriental Silicas Corporation) to give 226.8 g of water-absorbent resin particles (1). The water-absorbent resin particles (1) had a physiological saline absorption amount of 65 g/g, a physiological saline retention amount of 43 g/g, and a physiological saline absorption amount under a load of 4.14 kPa of 14 mL/g.

<Comparative Example 1>

The same procedures as in Example 1 were followed except that in Example 1, 4.42 g of a 0.5% by mass aqueous solution of ethylenediamine-N,N'-disuccinic acid-trisodium salt was not used, and the amount of water removed out of the system by azeotropic distillation of the n-heptane and water in [Surface-Crosslinking Step] was changed from 84.7 g to 80.3 g, to give 224.0 g of water-absorbent resin particles (2). The water content in the hydrous gel particles when adding the aqueous solution of sodium sulfite was 62% by mass, and the water content in the hydrous gel particles when adding the aqueous solution of L(+)-ascorbic acid was 63% by mass. The water-absorbent resin particles (2) had a physiological saline absorption amount of 62 g/g, a physiological saline retention amount of 42 g/g, and a physiological saline absorption amount under a load of 4.14 kPa of 18 mL/g.

<Comparative Example 2>

The same procedures as in Example 1 were followed except that in Example 1, a 20% by mass aqueous solution of sodium sulfite was not used, and the amount of water removed out of the system by azeotropic distillation of the n-heptane and water in [Surface-Crosslinking Step] was changed from 84.7 g to 83.0 g, to give 225.5 g of water-absorbent resin particles (3). The water content in the hydrous gel particles when adding the aqueous solution of ethylenediamine-N,N'-disuccinic acid-trisodium salt was 62% by mass, and the water content in the hydrous gel particles when adding the aqueous solution of L(+)-ascorbic acid was 64% by mass. The water-absorbent resin particles (3) had a physiological saline absorption amount of 61 g/g, a physiological saline retention amount of 37 g/g, and a physiological saline absorption amount under a load of 4.14 kPa of 18 mL/g.

<Comparative Example 3>

The same procedures as in Example 1 were followed except that in Example 1, a 0.1% by mass aqueous solution of L(+)-ascorbic acid was not used, and the amount of water removed out of the system by azeotropic distillation of the n-heptane and water in [Surface-Crosslinking Step] was changed from 84.7 g to 82.5 g, to give 224.8 g of water-absorbent resin particles (4). The water content in the hydrous gel particles when adding the aqueous solution of ethylenediamine-N,N'-disuccinic acid-trisodium salt was 62% by mass, and the water content in the hydrous gel particles when adding the aqueous solution of sodium sulfite was 64% by mass. The water-absorbent resin particles (4) had a physiological saline absorption amount of 73 g/g, a physiological saline retention amount of 42 g/g, and a physiological saline absorption amount under a load of 4.14 kPa of 16 mL/g.

<Example 2>

The same procedures as in Example 1 were followed except that in Example 1, the amount of water removed out of the system by azeotropic distillation of the n-heptane and water in the first step of adjusting the water content was changed from 175.1 g to 253.8 g, and the amount of water removed out of the system by azeotropic distillation of the n-heptane and water in [Surface-Crosslinking Step] was changed from 84.7 g to 6.1 g, to give 221.4 g of water-absorbent resin particles (5). The water content in the hydrous gel particles when adding the aqueous solution of ethylenediamine-N,N'-disuccinic acid-trisodium salt was 26% by mass, the water content in the hydrous gel particles when adding the aqueous solution of sodium sulfite was 28% by mass, and the water content in the hydrous gel particles when adding the aqueous solution of L(+)-ascorbic acid was 29% by mass. The water-absorbent resin particles (5) had a physiological saline absorption amount of 59 g/g, a physiological saline retention amount of 36 g/g, and a physiological saline absorption amount under a load of 4.14 kPa of 23 mL/g.

<Example 3>

The same procedures as in Example 1 were followed except that in Example 1, the amount of water removed out of the system by azeotropic distillation of the n-heptane and water in the first step of adjusting the water content was changed from 175.1 g to 223.2 g, and the amount of water removed out of the system by azeotropic distillation of the n-heptane and water in [Surface-Crosslinking Step] was changed from 84.7 g to 36.7 g, to give 223.8 g of water-absorbent resin particles (6). The water content in the hydrous gel particles when adding the aqueous solution of ethylenediamine-N,N'-disuccinic acid-trisodium salt was 40% by mass, the water content in the hydrous gel particles when adding the aqueous solution of sodium sulfite was 42% by mass, and the water content in the hydrous gel particles when adding the aqueous solution of L(+)-ascorbic acid was 43% by mass. The water-absorbent resin particles (6) had a physiological saline absorption amount of 66 g/g, a physiological saline retention amount of 43 g/g, and a physiological saline absorption amount under a load of 4.14 kPa of 12 mL/g.

<Example 4>

The same procedures as in Example 1 were followed except that in Example 1, the amount of water removed out of the system by azeotropic distillation of the n-heptane and water in the first step of adjusting the water content was changed from 175.1 g to 157.6 g, and the amount of water removed out of the system by azeotropic distillation of the n-heptane and water in [Surface-Crosslinking Step] was changed from 84.7 g to 102.2 g, to give 225.1 g of water-absorbent resin particles (7). The water content in the hydrous gel particles when adding the aqueous solution of ethylenediamine-N,N'-disuccinic acid-trisodium salt was 70% by mass, the water content in the hydrous gel particles when adding the aqueous solution of sodium sulfite was 72% by mass, and the water content in the hydrous gel particles when adding the aqueous solution of L(+)-ascorbic acid was 73% by mass. The water-absorbent resin particles (7) had a physiological saline absorption amount of 64 g/g, a physiological saline retention amount of 42 g/g, and a physiological saline absorption amount under a load of 4.14 kPa of 12 mL/g.

<Comparative Example 4>

The same procedures as in Example 1 were followed except that in Example 1, the amount of water removed out of the system by azeotropic distillation of the n-heptane and water in the first step of adjusting the water content was changed from 175.1 g to 135.8 g, and the amount of water removed out of the system by azeotropic distillation of the n-heptane and water in [Surface-Crosslinking Step] was changed from 84.7 g to 124.1 g, to give 223.8 g of water-absorbent resin particles (8). The water content in the hydrous gel particles when adding the aqueous solution of ethylenediamine-N,N'-disuccinic acid-trisodium salt was 80% by mass, the water content in the hydrous gel particles when adding the aqueous solution of sodium sulfite was 82% by mass, and the water content in the hydrous gel particles when adding the aqueous solution of L(+)-ascorbic acid was 83% by mass. The water-absorbent resin particles (8) had a physiological saline absorption amount of 67 g/g, a physiological saline retention amount of 46 g/g, and a physiological saline absorption amount under a load of 4.14 kPa of 12 mL/g.

<Comparative Example 5>

[Step of Producing Hydrous Gel Particles (Step 1)]

Hydrous gel particles were obtained following the same procedures as in [Step of Producing Hydrous Gel Particles] of Example 1.

[Step of Adding Additives to Hydrous Gel Particles (Step 2)]

After the hydrous gel particles were obtained as above, the flask was immersed in an oil bath set at 125°C to remove 135.8 g of water out of the system by azeotropic distillation of the n-heptane and water, while refluxing the n-heptane (first step of adjusting the water content). Then, 2.21 g of a 20% by mass aqueous solution of sodium sulfite was added with stirring. Here, the water content in the hydrous gel particles when adding the aqueous solution of sodium sulfite was 80% by mass. Then again, 89.2 g of water was removed out of the system by azeotropic distillation of the n-heptane and water, while refluxing the n-heptane (second step of adjusting the water content). Then, 4.42 g of a 0.5% by mass aqueous solution of ethylenediamine-N,N'-disuccinic acid-trisodium salt was added with stirring. Here, the water content in the hydrous gel particles when adding the aqueous solution of ethylenediamine-N,N'-disuccinic acid-trisodium salt was 40% by mass. Subsequently, 2.21 g of a 0.2% by mass aqueous solution of L(+)-ascorbic acid was added with stirring. Here, the water content in the hydrous gel particles when adding the aqueous solution of L(+)-ascorbic acid was 42% by mass.

[Surface-Crosslinking Step (Step 3)]

Then again, 34.9 g of water was removed out of the system by azeotropic distillation of the n-heptane and water, while refluxing the n-heptane. Then, 4.42 g (0.507 mmol) of a 2% by mass aqueous solution of ethylene glycol diglycidyl ether was added to the flask as a surface-crosslinking agent, and the flask was maintained at 83°C for 2 hours. Then, the n-heptane was evaporated at 125°C to dry the reaction mixture, and the resulting product was subsequently passed through a sieve with a mesh size of 850 μm to give 221.1 g of water-absorbent resin particles.

[Step of Adding Additives to Water-Absorbent Resin Particles]

Following the same procedures as in [Step of Adding Additives to Water-Absorbent Resin Particles] of Example 1, 222.2 g of water-absorbent resin particles (9) were obtained. The water-absorbent resin particles (9) had a physiological saline absorption amount of 65 g/g, a physiological saline retention amount of 43 g/g, and a physiological saline absorption amount under a load of 4.14 kPa of 14 mL/g.

<Example 5>

[Step of Producing Hydrous Gel Particles (Step 1)]

Hydrous gel particles were obtained following the same procedures as in [Step of Producing Hydrous Gel Particles] of Example 1.

[Step of Adding Additives to Hydrous Gel Particles (Step 2)]

After the hydrous gel particles were obtained as above, the flask was immersed in an oil bath set at 125°C to remove 135.8 g of water out of the system by azeotropic distillation of the n-heptane and water, while refluxing the n-heptane (first step of adjusting the water content). Then, 4.42 g of a 0.5% by mass aqueous solution of ethylenediamine-N,N'-disuccinic acid-trisodium salt was added with stirring. Here, the water content in the hydrous gel particles when adding the aqueous solution of ethylenediamine-N,N'-disuccinic acid-trisodium salt was 80% by mass. Then again, 91.8 g of water was removed out of the system by azeotropic distillation of the n-heptane and water, while refluxing the n-heptane (second step of adjusting the water content). Then, 2.21 g of a 20% by mass aqueous solution of sodium sulfite was added with stirring. Here, the water content in the hydrous gel particles when adding the aqueous solution of sodium sulfite was 40% by mass. Subsequently, 2.21 g of a 0.2% by mass aqueous solution of L(+)-ascorbic acid was added with stirring. Here, the water content in the hydrous gel particles when adding the aqueous solution of L(+)-ascorbic acid was 41% by mass.

[Surface-Crosslinking Step (Step 3)]

Then again, 32.3 g of water was removed out of the system by azeotropic distillation of the n-heptane and water, while refluxing the n-heptane. Then, 4.42 g (0.507 mmol) of a 2% by mass aqueous solution of ethylene glycol diglycidyl ether was added to the flask as a surface-crosslinking agent, and the flask was maintained at 83°C for 2 hours. Then, the n-heptane was evaporated at 125°C to dry the reaction mixture, and the resulting product was subsequently passed through a sieve with a mesh size of 850 μm to give 222.7 g of water-absorbent resin particles.

[Step of Adding Additives to Water-Absorbent Resin Particles]

Following the same procedures as in [Step of Adding Additives to Water-Absorbent Resin Particles] of Example 1, 223.8 g of water-absorbent resin particles (10) were obtained. The water-absorbent resin particles (10) had a physiological saline absorption amount of 65 g/g, a physiological saline retention amount of 41 g/g, and a physiological saline absorption amount under a load of 4.14 kPa of 20 mL/g.

<Example 6>

[Step of Producing Hydrous Gel Particles (Step 1)]

Hydrous gel particles were obtained following the same procedures as in [Step of Producing Hydrous Gel Particles] of Example 1.

[Step of Adding Additives to Hydrous Gel Particles (Step 2)]

After the hydrous gel particles were obtained as above, the flask was immersed in an oil bath set at 125°C to remove 135.8 g of water out of the system by azeotropic distillation of the n-heptane and water, while refluxing the n-heptane (first step of adjusting the water content). Then, 2.21 g of a 0.2% by mass aqueous solution of L(+)-ascorbic acid was added with stirring. Here, the water content in the hydrous gel particles when adding the aqueous solution of L(+)-ascorbic acid was 80% by mass. Then again, 89.6 g of water was removed out of the system by azeotropic distillation of the n-heptane and water, while refluxing the n-heptane (second step of adjusting the water content). Then, 4.42 g of a 0.5% by mass aqueous solution of ethylenediamine-N,N'-disuccinic acid-trisodium salt was added with stirring. Here, the water content in the hydrous gel particles when adding the aqueous solution of ethylenediamine-N,N'-disuccinic acid-trisodium salt was 40% by mass. Subsequently, 2.21 g of a 20% by mass aqueous solution of sodium sulfite was added with stirring. Here, the water content in the hydrous gel particles when adding the aqueous solution of sodium sulfite was 42% by mass.

[Surface-Crosslinking Step (Step 3)]

Then again, 34.5 g of water was removed out of the system by azeotropic distillation of the n-heptane and water, while refluxing the n-heptane. Then, 4.42 g (0.507 mmol) of a 2% by mass aqueous solution of ethylene glycol diglycidyl ether was added to the flask as a surface-crosslinking agent, and the flask was maintained at 83°C for 2 hours. Then, the n-heptane was evaporated at 125°C to dry the reaction mixture, and the resulting product was subsequently passed through a sieve with a mesh size of 850 μm to give 223.7 g of water-absorbent resin particles.

[Step of Adding Additives to Water-Absorbent Resin Particles]

Following the same procedures as in [Step of Adding Additives to Water-Absorbent Resin Particles] of Example 1, 224.8 g of water-absorbent resin particles (11) were obtained. The water-absorbent resin particles (11) had a physiological saline absorption amount of 59 g/g, a physiological saline retention amount of 39 g/g, and a physiological saline absorption amount under a load of 4.14 kPa of 17 mL/g.

<Comparative Example 6>

[Step of Producing Hydrous Gel Particles (Step 1)]

Hydrous gel particles were obtained following the same procedures as in [Step of Producing Hydrous Gel Particles] of Example 1.

[Surface-Crosslinking Step (Step 3)]

After the hydrous gel particles were obtained as above, the flask was immersed in an oil bath set at 125°C to remove 251.5 g of water out of the system by azeotropic distillation of the n-heptane and water, while refluxing the n-heptane. Then, 4.42 g (0.507 mmol) of a 2% by mass aqueous solution of ethylene glycol diglycidyl ether was added to the flask as a surface-crosslinking agent, and the flask was maintained at 83°C for 2 hours. Then, the n-heptane was evaporated at 125°C to dry the reaction mixture, and the resulting product was subsequently passed through a sieve with a mesh size of 850 μm to give 217.6 g of water-absorbent resin particles.

[Step of Adding Additives to Water-Absorbent Resin Particles]

To 100 parts by mass of the water-absorbent resin particles obtained in [Surface-Crosslinking Step] above, 0.7 parts by mass of diethylenetriaminepentaacetic acid (DTPA·5H), 2 parts by mass of sodium sulfite, and 0.02 parts by mass of L(+)-ascorbic acid were added as powders and then mixed for 30 minutes using a cross-rotary mixer from Meiwa Kogyo Co., Ltd. (conditions: revolution speed 50 rpm, rotation speed 50 rpm), to give a mixture. Additionally, 100 parts by mass of the obtained mixture was mixed with 0.5 parts by mass of amorphous silica (Tokusil NP-S from Oriental Silicas Corporation) to give water-absorbent resin particles (12). The water-absorbent resin particles (12) had a physiological saline absorption amount of 60 g/g, a physiological saline retention amount of 42 g/g, and a physiological saline absorption amount under a load of 4.14 kPa of 14 mL/g.

<Comparative Example 7>

[Step of Producing Hydrous Gel Particles (Step 1)]

Hydrous gel particles were obtained following the same procedures as in [Step of Producing Hydrous Gel Particles] of Example 1.

[Surface-Crosslinking Step (Step 3)]

After the hydrous gel particles were obtained as above, the flask was immersed in an oil bath set at 125°C to remove 251.5 g of water out of the system by azeotropic distillation of the n-heptane and water, while refluxing the n-heptane. Then, 4.42 g (0.507 mmol) of a 2% by mass aqueous solution of ethylene glycol diglycidyl ether was added to the flask as a surface-crosslinking agent, and the flask was maintained at 83°C for 2 hours. Then, the n-heptane was evaporated at 125°C to dry the reaction mixture, and the resulting product was subsequently passed through a sieve with a mesh size of 850 μm to give 226.8 g of water-absorbent resin particles.

[Step of Adding Additives to Water-Absorbent Resin Particles]

20 g of the water-absorbent resin particles obtained in [Surface-Crosslinking Step] above was weighed into an 11 cm inner diameter, cylindrical round-bottomed separable flask equipped with an anchor-type stirring blade made of a fluororesin. Subsequently, 0.2 g of a 1.0% by mass aqueous solution of diethylenetriaminepentaacetic acid·pentasodium salt (DTPA·5Na) was dropped into the separable flask with a pasteur pipette while stirring at 300 rpm, and then stirred for 10 minutes to give a mixture. This mixture was heated at 100°C for 30 minutes; thereafter, 0.2 parts by mass of sodium sulfite and 0.002 parts by mass of L(+)-ascorbic acid were added as powders to 100 parts by mass of the mixture after heating and then mixed for 30 minutes using a cross-rotary mixer from Meiwa Kogyo Co., Ltd. (conditions: revolution speed 50 rpm, rotation speed 50 rpm), to give a mixture. Additionally, 100 parts by mass of the obtained mixture was mixed with 0.5 parts by mass of amorphous silica (Tokusil NP-S from Oriental Silicas Corporation) to give water-absorbent resin particles. The water-absorbent resin particles had a physiological saline absorption amount of 58 g/g, a physiological saline retention amount of 40 g/g, and a physiological saline absorption amount under a load of 4.14 kPa of 21 mL/g.

<Comparative Example 8>

The same procedures as in Comparative Example 7 were followed except that in Comparative Example 7, 0.2 g of a 1.0% by mass aqueous solution of ethylenediamine-N,N'-disuccinic acid-trisodium salt (EDDS·3Na) was used instead of 0.2 g of a 1.0% by mass aqueous solution of diethylenetriaminepentaacetic acid-pentasodium salt (DTPA·5Na), to give 218.8 g of water-absorbent resin particles (14). The water-absorbent resin particles (14) had a physiological saline absorption amount of 56 g/g, a physiological saline retention amount of 39 g/g, and a physiological saline absorption amount under a load of 4.14 kPa of 23 mL/g.

<Example 7>

The same procedures as in Example 1 were followed except that in Example 1, 4.42 g of a 0.5% by mass aqueous solution of diethylenetriaminepentaacetic acid-pentasodium salt (DTPA·5Na) was used instead of 4.42 g of a 0.5% by mass aqueous solution of ethylenediamine-N,N'-disuccinic acid-trisodium salt, to give 225.6 g of water-absorbent resin particles (15). The water content in the hydrous gel particles when adding the aqueous solution of diethylenetriaminepentaacetic acid·pentasodium salt was 62% by mass, the water content in the hydrous gel particles when adding the aqueous solution of sodium sulfite was 64% by mass, and the water content in the hydrous gel particles when adding the aqueous solution of L(+)-ascorbic acid was 65% by mass. The water-absorbent resin particles (15) had a physiological saline absorption amount of 58 g/g, a physiological saline retention amount of 39 g/g, and a physiological saline absorption amount under a load of 4.14 kPa of 14 mL/g.

<Example 8>

The same procedures as in Example 1 were followed except that in Example 1, 4.42 g of a 0.5% by mass aqueous solution of ethylenediamine tetramethylenephosphonic acid (EDTMP·8H) was used instead of 4.42 g of a 0.5% by mass aqueous solution of ethylenediamine-N,N'-disuccinic acid-trisodium salt, to give 224.6 g of water-absorbent resin particles (16). The water content in the hydrous gel particles when adding the aqueous solution of ethylenediamine tetramethylenephosphonic acid was 62% by mass, the water content in the hydrous gel particles when adding the aqueous solution of sodium sulfite was 64% by mass, and the water content in the hydrous gel particles when adding the aqueous solution of L(+)-ascorbic acid was 65% by mass. The water-absorbent resin particles (16) had a physiological saline absorption amount of 61 g/g, a physiological saline retention amount of 37 g/g, and a physiological saline absorption amount under a load of 4.14 kPa of 18 mL/g.

<Example 9>

[Step of Producing Hydrous Gel Particles (Step 1)]

Hydrous gel particles were obtained following the same procedures as in [Step of Producing Hydrous Gel Particles] of Example 1.

[Step of Adding Additives to Hydrous Gel Particles (Step 2)]

After the hydrous gel particles were obtained as above, the flask was immersed in an oil bath set at 125°C to remove 175.1 g of water out of the system by azeotropic distillation of the n-heptane and water, while refluxing the n-heptane (first step of adjusting the water content). Then, 4.42 g of a 0.5% by mass aqueous solution of ethylenediamine-N,N'-disuccinic acid-trisodium salt was added with stirring. Here, the water content in the hydrous gel particles when adding the aqueous solution of ethylenediamine-N,N'-disuccinic acid-trisodium salt was 62% by mass. Then again, 15.3 g of water was removed out of the system by azeotropic distillation of the n-heptane and water, while refluxing the n-heptane (second step of adjusting the water content). Then, 2.21 g of a 20% by mass aqueous solution of sodium sulfite was added with stirring. Here, the water content in the hydrous gel particles when adding the aqueous solution of sodium sulfite was 57% by mass. Then again, 12.7 g of water was removed out of the system by azeotropic distillation of the n-heptane and water, while refluxing the n-heptane (third step of adjusting the water content). Subsequently, 2.21 g of a 0.2% by mass aqueous solution of L(+)-ascorbic acid was added with stirring. Here, the water content in the hydrous gel particles when adding the aqueous solution of L(+)-ascorbic acid was 52% by mass.

[Surface-Crosslinking Step (Step 3)]

Then again, 56.7 g of water was removed out of the system by azeotropic distillation of the n-heptane and water, while refluxing the n-heptane. Then, 4.42 g (0.507 mmol) of a 2% by mass aqueous solution of ethylene glycol diglycidyl ether was added to the flask as a surface-crosslinking agent, and the flask was maintained at 83°C for 2 hours. Then, the n-heptane was evaporated at 125°C to dry the reaction mixture, and the resulting product was subsequently passed through a sieve with a mesh size of 850 μm to give 219.7 g of water-absorbent resin particles.

[Step of Adding Additives to Water-Absorbent Resin Particles]

Following the same procedures as in [Step of Adding Additives to Water-Absorbent Resin Particles] of Example 1, 220.8 g of water-absorbent resin particles (17) were obtained. The water-absorbent resin particles (17) had a physiological saline absorption amount of 64 g/g, a physiological saline retention amount of 42 g/g, and a physiological saline absorption amount under a load of 4.14 kPa of 16 mL/g.

<Example 10>

The same procedures as in Example 9 were followed except that in Example 9, 8.83 g of a 0.5% by mass aqueous solution of ethylenediamine-N,N'-disuccinic acid-trisodium salt was used instead of 4.42 g of a 0.5% by mass solution of ethylenediamine-N,N'-disuccinic acid-trisodium salt, and the amount of water removed out of the system by azeotropic distillation of the n-heptane and water in the second step of adjusting the water content was changed from 15.3 g to 19.7 g, to give 225.7 g of water-absorbent resin particles (18). The water content in the hydrous gel particles when adding the aqueous solution of ethylenediamine-N,N'-disuccinic acid-trisodium salt was 62% by mass, the water content in the hydrous gel particles when adding the aqueous solution of sodium sulfite was 57% by mass, and the water content in the hydrous gel particles when adding the aqueous solution of L(+)-ascorbic acid was 52% by mass. The water-absorbent resin particles (18) had a physiological saline absorption amount of 64 g/g, a physiological saline retention amount of 40 g/g, and a physiological saline absorption amount under a load of 4.14 kPa of 11 mL/g.

<Example 11>

The same procedures as in Example 9 were followed except that in Example 9, 2.21 g of a 0.5% by mass aqueous solution of ethylenediamine-N,N'-disuccinic acid-trisodium salt was used instead of 4.42 g of a 0.5% by mass solution of ethylenediamine-N,N'-disuccinic acid-trisodium salt, and the amount of water removed out of the system by azeotropic distillation of the n-heptane and water in the second step of adjusting the water content was changed from 15.3 g to 13.1 g, to give 223.4 g of water-absorbent resin particles (19). The water content in the hydrous gel particles when adding the aqueous solution of ethylenediamine-N,N'-disuccinic acid-trisodium salt was 62% by mass, the water content in the hydrous gel particles when adding the aqueous solution of sodium sulfite was 57% by mass, and the water content in the hydrous gel particles when adding the aqueous solution of L(+)-ascorbic acid was 52% by mass. The water-absorbent resin particles (19) had a physiological saline absorption amount of 62 g/g, a physiological saline retention amount of 46 g/g, and a physiological saline absorption amount under a load of 4.14 kPa of 10 mL/g.

<Example 12>

The same procedures as in Example 9 were followed except that in Example 9, 3.31 g of a 20% by mass aqueous solution of sodium sulfite was used instead of 2.21 g of a 20% by mass aqueous solution of sodium sulfite, and the amount of water removed out of the system by azeotropic distillation of the n-heptane and water in the third step of adjusting the water content was changed from 12.7 g to 13.6 g, to give 223.8 g of water-absorbent resin particles (20). The water content in the hydrous gel particles when adding the aqueous solution of ethylenediamine-N,N'-disuccinic acid-trisodium salt was 62% by mass, the water content in the hydrous gel particles when adding the aqueous solution of sodium sulfite was 57% by mass, and the water content in the hydrous gel particles when adding the aqueous solution of L(+)-ascorbic acid was 52% by mass. The water-absorbent resin particles (20) had a physiological saline absorption amount of 67 g/g, a physiological saline retention amount of 50 g/g, and a physiological saline absorption amount under a load of 4.14 kPa of 8 mL/g.

<Example 13>

The same procedures as in Example 9 were followed except that in Example 9, 1.10 g of a 20% by mass aqueous solution of sodium sulfite was used instead of 2.21 g of a 20% by mass aqueous solution of sodium sulfite, and the amount of water removed out of the system by azeotropic distillation of the n-heptane and water in the third step of adjusting the water content was changed from 12.7 g to 11.8 g, to give 224.6 g of water-absorbent resin particles (21). The water content in the hydrous gel particles when adding the aqueous solution of ethylenediamine-N,N'-disuccinic acid-trisodium salt was 62% by mass, the water content in the hydrous gel particles when adding the aqueous solution of sodium sulfite was 57% by mass, and the water content in the hydrous gel particles when adding the aqueous solution of L(+)-ascorbic acid was 52% by mass. The water-absorbent resin particles (21) had a physiological saline absorption amount of 65 g/g, a physiological saline retention amount of 45 g/g, and a physiological saline absorption amount under a load of 4.14 kPa of 11 mL/g.

<Example 14>

The same procedures as in Example 9 were followed except that in Example 9, 11.0 g of a 0.2% by mass aqueous solution of L(+)-ascorbic acid was used instead of 2.21 g of a 0.2% by mass aqueous solution of L(+)-ascorbic acid, and the amount of water removed out of the system by azeotropic distillation of the n-heptane and water in [Surface-Crosslinking Step] was changed from 56.7 g to 65.5 g, to give 226.8 g of water-absorbent resin particles (22). The water content in the hydrous gel particles when adding the aqueous solution of ethylenediamine-N,N'-disuccinic acid-trisodium salt was 62% by mass, the water content in the hydrous gel particles when adding the aqueous solution of sodium sulfite was 57% by mass, and the water content in the hydrous gel particles when adding the aqueous solution of L(+)-ascorbic acid was 52% by mass. The water-absorbent resin particles (22) had a physiological saline absorption amount of 62 g/g, a physiological saline retention amount of 45 g/g, and a physiological saline absorption amount under a load of 4.14 kPa of 22 mL/g.

<Example 15>

The same procedures as in Example 9 were followed except that in Example 9, 1.10 g of a 0.2% by mass aqueous solution of L(+)-ascorbic acid was used instead of 2.21 g of a 0.2% by mass aqueous solution of L(+)-ascorbic acid, and the amount of water removed out of the system by azeotropic distillation of the n-heptane and water in [Surface-Crosslinking Step] was changed from 56.7 g to 55.6 g, to give 224.0 g of water-absorbent resin particles (23). The water content in the hydrous gel particles when adding the aqueous solution of ethylenediamine-N,N'-disuccinic acid-trisodium salt was 62% by mass, the water content in the hydrous gel particles when adding the aqueous solution of sodium sulfite was 57% by mass, and the water content in the hydrous gel particles when adding the aqueous solution of L(+)-ascorbic acid was 52% by mass. The water-absorbent resin particles (23) had a physiological saline absorption amount of 66 g/g, a physiological saline retention amount of 46 g/g, and a physiological saline absorption amount under a load of 4.14 kPa of 10 mL/g.

<Water Content in Hydrous Gel Particles>

The water content in the hydrous gel particles was calculated as follows. Using an amount of water W1 (g) contained in the aqueous solution used to produce the hydrous gel particles in [Step of Producing Hydrous Gel Particles (Step 1)], an amount of water W2 (g) contained in the aqueous solutions of the additives added to the system in [Step of Adding Additives to Hydrous Gel Particles (Step 2)], an amount of water W3 (g) removed out of the system by azeotropic distillation of the n-heptane and water in [Step of Adding Additives to Hydrous Gel Particles (Step 2)], an amount of water W4 (g) removed out of the system by azeotropic distillation of the n-heptane and water in [Surface-Crosslinking Step (Step 3)], and an amount M1 (g) of the water-soluble ethylenically unsaturated monomer contained in the aqueous solution used to produce the hydrous gel particles in [Step of Producing Hydrous Gel Particles (Step 1)], the water content in the hydrous gel particles was calculated based on the following equation:

Water content (% by mass) in hydrous gel particles = $(W1 + W2 - W3 - W4) \times 100/M1$

<Physiological Saline Retention Amount>

500 g of a 0.9% by mass aqueous solution of sodium chloride (physiological saline) was weighed into a 500 mL beaker, and 2.0 g of the water-absorbent resin particles were dispersed so as not to form unswollen lumps while stirring with a 3 cm stirrer bar (without a ring) at 600 rpm. The water-absorbent resin particles were sufficiently swollen by being allowed to stand for 60 minutes while stirring. Then, a standard sieve with mesh size of 75 μm, whose mass Wa (g) had been previously measured, was used to filter the beaker contents, and excess water was filtered off by inclining the sieve at an inclination angle of about 30 degrees with respect to the horizontal direction, and allowing the sieve to stand for 30 minutes. A mass Wb (g) of the sieve containing the swollen gel was measured, and the physiological saline absorption capacity was calculated based on the following equation:

$$\text{Physiological saline retention amount (g/g)} = [Wb - Wa]/2.0$$

<Physiological Saline Retention Amount>

2.0 g of the water-absorbent resin particles were weighed into a cotton bag (Cottonbroad No. 60, 100 mm in width $\times$ 200 mm in length), and the cotton bag was placed in a 500 mL beaker. 500 g of a 0.9% by mass aqueous solution of sodium chloride (physiological saline) was poured at once into the cotton bag containing the water-absorbent resin particles so as to prevent formation of unswollen lumps, then the top of the cotton bag was closed with a rubber band, and the cotton bag was allowed to stand for 30 minutes to cause the water-absorbent resin particles to swell. After 30 minutes, the cotton bag was dehydrated for 1 minute using a dehydrator (product number: H-122 from Kokusan Co., Ltd.) set at a centrifugal force of 167 G, and a mass Wd (g) of the cotton bag containing the swollen gel after dehydration was measured. The same operation was performed without adding the water-absorbent resin particles, and an empty mass We (g) of the cotton bag upon wetting was measured. The physiological saline retention amount was calculated according to the following equation:

$$\text{Physiological saline retention amount (g/g)} = [Wd - We]/2.0$$

<Physiological Saline Absorption Amount Under a Load of 4.14 kPa>

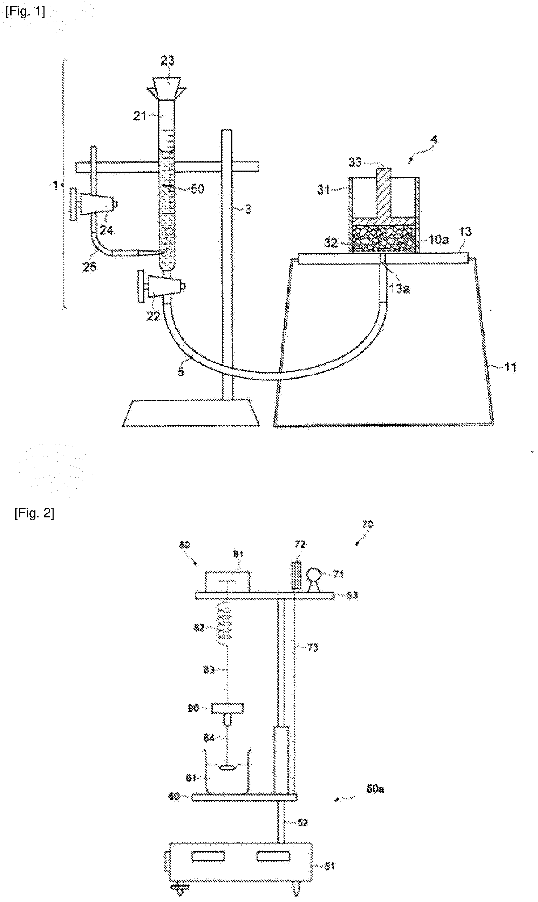

Physiological saline absorption amount under a load of 4.14 kPa (water absorption amount under load) was measured using the measurement apparatus as schematically shown in Fig. 1. Two measurements were made for one type of water-absorbent resin particles, and the average value was obtained. The measurement apparatus includes a burette unit 1, a clamp 3, a conduit 5, a stand 11, a measurement table 13, and a measurement unit 4 placed on the measurement table 13. The burette unit 1 has a graduated burette tube 21, a rubber stopper 23 for sealing the upper opening of the burette tube 21, a cock 22 connected to the lower end of the burette tube 21, an air inlet tube 25 connected to a lower portion of the burette tube 21, and a cock 24. The burette unit 1 is fixed by the clamp 3. The flat measurement table 13 has a through hole 13a with a diameter of 2 mm formed in the central portion thereof, and is supported by the variable-height stand 11. The through hole 13a of the measurement table 13 and the cock 22 of the burette unit 1 are connected through the conduit 5. The conduit 5 has an inner diameter of 6 mm.
The measurement unit 4 includes a cylinder 31 made of plexiglas, a polyamide mesh 32 bonded to one opening of the cylinder 31, and a weight 33 vertically movable within the cylinder 31. The cylinder 31 is placed on the measurement table 13 with the polyamide mesh 32 therebetween. The cylinder 31 has an inner diameter of 20 mm. The polyamide mesh 32 has a mesh size of 75 $\mu$m (200 mesh). The weight 33 has a diameter of 19 mm and a mass of 119.6 g and, as described later, can apply a load of 4.14 kPa (0.6 psi) to water-absorbent resin particles 10a uniformly disposed on the polyamide mesh 32.
First, the cock 22 and the cock 24 of the burette unit 1 were closed, and 0.9% by mass physiological saline adjusted to 25°C was placed in the burette tube 21 through the upper opening of the burette tube 21. Subsequently, the upper opening of the burette tube 21 was sealed with the rubber stopper 23, and then the cock 22 and the cock 24 were opened. The conduit 5 was filled with the 0.9% by mass saline 50 to prevent air bubbles from entering. The height of the measurement table 13 was adjusted so that the height of the surface of the 0.9% by mass saline reached into the through hole 13a was equal to the height of the upper surface of the measurement table 13. After the adjustment, the height of the surface of the 0.9% by mass saline 50 in the burette tube 21 was read on the graduations of the burette tube 21, and this position was determined as a zero point (reading at zero seconds).
In the measurement unit 4, 0.10 g of the water-absorbent resin particles 10a were uniformly disposed on the polyamide mesh 32 in the cylinder 31, the weight 33 was disposed on the water-absorbent resin particles 10a, and the cylinder 31 was installed such that the central portion thereof aligned with the conduit opening at the central portion of the measurement table 13. An amount of reduction Wc (mL) in the physiological saline in the burette tube 21 at 60 minutes after the beginning of absorption of the physiological saline by the water-absorbent resin particles 10a through the conduit 5 (that is, the amount of the physiological saline absorbed by the water-absorbent resin particles 10a) was read, and the physiological saline absorption capacity under a load of 4.14 kPa of the water-absorbent resin particles 10a was calculated based on the following equation:

Physiological saline absorption capacity (mL/g) under a load of 4.14 kPa = Wc (mL)/mass (g) of the water-absorbent resin particles

<Loss on Drying>

2.0 g of the water-absorbent resin particles were placed in an aluminum foil case (No.8) previously adjusted to a constant weight (W1 (g)), the opening of the aluminum foil case was lightly closed, and a total mass W2 (g) of the aluminum foil case containing this sample was accurately weighed. The aluminum foil case containing the sample was dried for 2 hours in a hot-air dryer (from ADVANTEC, model: FV-320) set at an internal temperature of 105°C. The aluminum foil case containing the sample after drying was allowed to cool to room temperature in a desiccator. A total mass W3 (g) of the aluminum foil case containing the sample after cooling was measured. The loss on drying of the sample was calculated based on the following equation:

$$\text{Loss on drying [\% by mass]} = [\{(W2 - W1) - (W3 - W1)\}/(W2 - W1)] \times 100$$

<Yellowing Test under High Temperature and High Humidity (Measurement of Yellowness Index)>

2.0 g of the water-absorbent resin particles were placed in a glass measurement container with an inner diameter of 3 cm. The yellowness index of the water-absorbent resin particles was measured with a colorimeter (Color Meter ZE6000 from Nippon Denshoku Industries Co., Ltd.) having the tristimulus values X, Y, and Z of a colorimetric color difference meter calibrated with a standard white plate. From the obtained X, Y, and Z (tristimulus values) of the water-absorbent resin particles, the yellowness index was calculated based on the following equation and used as an initial value.

$$\text{Yellowness index} = 100 \, (1.28X - 1.06Z)/Y$$

Coloration of the water-absorbent resin particles with time was tested as follows. 2.0 g of the water-absorbent resin particles were placed uniformly in a glass petri dish with an inner diameter of 3 cm and a depth of 1 cm, and the container was stored for a predetermined number of days (7 days or 14 days) in a constant temperature and humidity machine (LHU-113 from Espec Corporation) set at a temperature of $70 \pm 2°C$ and a relative humidity of $90 \pm 2\%$. Then, the container was removed from the constant temperature and humidity chamber and allowed to cool to room temperature for a while. The entire amount of the water-absorbent resin particles in the container was placed in a glass measurement container with an inner diameter of 3 cm, and the yellowness index of the water-absorbent resin particles was measured with a colorimeter (Color Meter ZE6000 from Nippon Denshoku Industries Co., Ltd.). From the obtained X, Y, and Z (tristimulus values) of the water-absorbent resin particles, the yellowness index was calculated based on the following equation:

$$\text{Yellowness index} = 100 \, (1.28X - 1.06Z)/Y$$

<Test on gel after Absorption of Artificial Urine>

Using artificial urine, a gel in which the water-absorbent resin particles had absorbed water (swollen gel) was tested.

(Preparation of Artificial Urine)

Artificial urine of the following composition was prepared.
Urea: 20.0 g
Sodium chloride: 8.0 g
Calcium chloride dihydrate: 0.3 g
Magnesium sulfate heptahydrate: 0.8 g
L(+)-ascorbic acid: 0.2 g
Ion-exchanged water: 970.9 g

(Preparation of Swollen Gel)

39.0 g of the artificial urine was weighed into a 100 mL inner volume beaker, a magnetic stirrer bar (8 mm in diameter $\times$ 30 mm, without a ring) was introduced and placed on a magnetic stirrer (HS-30D from Iuchi Co., Ltd.), and then rotated at 600 rpm. Subsequently, 1.00 g of the water-absorbent resin particles were added into the beaker under stirring and stirred until the rotating vortex disappeared and the liquid surface became horizontal. In this manner, a swollen gel for use as a measurement sample was prepared. Immediately after the preparation of the swollen gel, the beaker containing the swollen gel was covered with a wrap (Diawrap from Mitsubishi Chemical Corporation).

(Measurement of Gel Strength)

The gel strength after each standing time at each temperature was measured using an apparatus having the measurement principle as shown in Fig. 2. The apparatus shown in Fig. 2 includes a support unit 50a, a movable mount plate 60, a driving unit 70 for driving the movable mount plate 60, and a measurement unit 80. In the support unit 50a, a stand 53 is fixed to an upper portion of a column 52 standing on a column mount 51. The movable mount plate 60 is attached to the column 52 to be vertically movable. A measurement sample (gel) 61 can be mounted on the movable mount plate 60. A pulse motor 71 is mounted on the stand 53, and rotating a pulley 72 causes the movable mount plate 60 to vertically move via a wire 73. In the measuring unit 80, a disk-equipped pressure-sensitive shaft 84 is attached to a load cell 81 for measuring a strain caused by deformation, via a precision spring 82 and a connecting shaft 83. The disk-equipped pressure-sensitive shaft 84 has a disk at its tip. Depending on the measurement conditions, the diameter of the disk can vary. A weight 90 can be mounted on an upper portion of the disk-equipped pressure-sensitive shaft 84. The operation principle of the apparatus for measuring gel strength is as follows. An upper portion of the precision spring 82 is fixed to the load cell 81 (stress detector), and a lower portion of the precision spring 82 is connected to the disk-equipped pressure-sensitive shaft 84 with the predetermined weight 90 mounted thereon and suspended vertically. The movable mount plate 60 on which the measurement sample 61 is placed is raised at a constant speed by the rotation of the pulse motor 71. A load is applied at a constant speed to the measurement sample 61 via the precision spring 82, the strain caused by deformation is measured by the load cell 81, and the hardness is measured and calculated. The gel strength value ($N/m^2$) was measured using Curdmeter-MAX (product number: ME-500 from Asuka Kiki Co., Ltd.), using a 16 mm diameter disk for the disk-equipped pressure-sensitive shaft 84, a load of 400 g, a speed of 7 seconds/inch, and the viscous mode setting, under the conditions of temperature and standing time as described in each of (Initial Value of Gel Strength) and (Gel Strength after Standing at 37°C for 14 Hours) below.

(Initial Value of Gel Strength)

The beaker containing the swollen gel covered with the wrap was allowed to stand for 60 minutes in an environment at a temperature of 25 $\pm$ 2°C and a relative humidity of 50 $\pm$ 10%, then the wrap was removed from the beaker containing the swollen gel, and the gel strength was measured by the above-described method. This value of gel strength was determined as the gel strength (initial value).

(Gel Strength after Standing at 37°C for 14 Hours)

The beaker containing the swollen gel covered with the wrap was allowed to stand for 14 hours in a constant temperature and humidity machine (LHU-113 from Espec Corporation) set at a temperature of 37 $\pm$ 2°C and a relative humidity of 60 $\pm$ 10%, then the wrap was removed from the beaker containing the swollen gel, and the gel strength was measured by the above-described method. This value of gel strength was determined as the gel strength (14-hour value).

[Table 1]

| Table 1 | Additives | | | | | | | | | | Properties of water-absorbent resin particles | | | | | | | | |
| | Chelating agent* | | | Sulfite-based compound | | | Organic antioxidant | | | | General performance | | | | Yellowing resistance (measurement of yellowness index) | | | Gel stability | |
| | Type | Amount added (vs. 100 parts by mass of monomer [part(s) by mass] | Water content [% by mass] during addition | Type | Amount added (vs. 100 parts by mass of monomer [part(s) by mass] | Water content [% by mass] during addition | Type | Amount added (vs. 100 parts by mass of monomer [part(s) by mass] | Amount added (vs. 100 parts by mass of sulfite-based compound [part(s) by mass] | Water content [% by mass] during addition | Physiological saline absorption amount [g/g] | Physiological saline retention amount [g/g] | Physiological saline absorption amount [mL/g] under a load of 4.14 kPa | Loss on drying [% by mass] | Initial value of yellowness index | Yellowness index after 7 days | Yellowness index after 14 days | Gel strength (initial value)[N/m²] | Gel strength (14-hour value) [N/m²] |
|---|---|---|---|---|---|---|---|---|---|---|---|---|---|---|---|---|---|---|---|
| Ex.1 | EDDS | 0.01 | 62 | Sodium sulfite | 0.20 | 64 | L(+)-Ascorbic acid | 0.002 | 1.00 | 65 | 65 | 43 | 14 | 6 | 7 | 22 | 33 | 6511 | 7044 |
| Comp. Ex.1 | — | — | — | Sodium sulfite | 0.20 | 62 | L(+)-Ascorbic acid | 0.002 | 1.00 | 63 | 62 | 42 | 18 | 7 | 3 | 16 | 34 | 7616 | 0 |
| Comp. Ex.2 | EDDS | 0.01 | 62 | — | — | — | L(+)-Ascorbic acid | 0.002 | — | 64 | 61 | 37 | 18 | 7 | 4 | 15 | 34 | 8125 | 5439 |
| Comp. Ex.3 | EDDS | 0.01 | 62 | Sodium sulfite | 0.20 | 64 | — | — | — | — | 73 | 42 | 16 | 7 | 7 | 22 | 29 | 6643 | 6510 |
| Ex.2 | EDDS | 0.01 | 26 | Sodium sulfite | 0.20 | 28 | L(+)-Ascorbic acid | 0.002 | 1.00 | 29 | 59 | 36 | 23 | 7 | 6 | 22 | 38 | 9829 | 6060 |
| Ex.3 | EDDS | 0.01 | 40 | Sodium sulfite | 0.20 | 42 | L(+)-Ascorbic acid | 0.002 | 1.00 | 43 | 66 | 43 | 12 | 6 | 4 | 19 | 34 | 8624 | 8470 |
| Ex.4 | EDDS | 0.01 | 70 | Sodium sulfite | 0.20 | 72 | L(+)-Ascorbic acid | 0.002 | 1.00 | 73 | 64 | 42 | 12 | 7 | 3 | 20 | 34 | 7893 | 7397 |
| Comp. Ex.4 | EDDS | 0.01 | 80 | Sodium sulfite | 0.20 | 82 | L(+)-Ascorbic acid | 0.002 | 1.00 | 83 | 67 | 46 | 12 | 7 | 6 | 20 | 36 | 6346 | 5215 |
| Comp. Ex.5 | EDDS | 0.01 | 40 | Sodium sulfite | 0.20 | 80 | L(+)-Ascorbic acid | 0.002 | 1.00 | 42 | 65 | 43 | 14 | 7 | 4 | 20 | 34 | 8191 | 4274 |
| Ex.5 | EDDS | 0.01 | 80 | Sodium sulfite | 0.20 | 40 | L(+)-Ascorbic acid | 0.002 | 1.00 | 41 | 65 | 41 | 20 | 7 | 6 | 23 | 38 | 8747 | 7019 |
| Ex.6 | EDDS | 0.01 | 40 | Sodium sulfite | 0.20 | 42 | L(+)-Ascorbic acid | 0.002 | 1.00 | 80 | 59 | 39 | 17 | 6 | 6 | 21 | 38 | 8258 | 8204 |
| Comp. Ex.6 | DTPA | 0.7(vs. water-absorbent resin particles) | Powder mixing (after surface-crosslinking) | Sodium sulfite | 2.0(vs. water-absorbent resin particles) | Powder mixing (after surface-crosslinking) | L(+)-Ascorbic acid | 0.02(vs. water-absorbent resin particles) | 1.00 | Powder mixing (after surface-crosslinking) | 60 | 42 | 14 | 7 | 6 | 54 | 55 | 8900 | 7919 |
| Comp. Ex.7 | DTPA | 0.01(vs. water-absorbent resin particles) | Dropwise addition of aqueous solution (after surface-crosslinking) | Sodium sulfite | 0.2(vs. water-absorbent resin particles) | Powder mixing (after surface-crosslinking) | L(+)-Ascorbic acid | 0.002(vs. water-absorbent resin particles) | 1.00 | Powder mixing (after surface-crosslinking) | 58 | 40 | 21 | 5 | 7 | 25 | 43 | 9931 | 10311 |
| Comp. Ex.8 | EDDS | 0.01(vs. water-absorbent resin particles) | Dropwise addition of aqueous solution (after surface-crosslinking) | Sodium sulfite | 0.2(vs. water-absorbent resin particles) | Powder mixing (after surface-crosslinking) | L(+)-Ascorbic acid | 0.002(vs. water-absorbent resin particles) | 1.00 | Powder mixing (after surface-crosslinking) | 56 | 39 | 23 | 5 | 7 | 33 | 48 | 8359 | 9997 |
| Ex.7 | DTPA | 0.01 | 62 | Sodium sulfite | 0.20 | 64 | L(+)-Ascorbic acid | 0.002 | 1.00 | 65 | 58 | 39 | 14 | 6 | 5 | 17 | 26 | 7125 | 6927 |
| Ex.8 | EDTMP | 0.01 | 62 | Sodium sulfite | 0.20 | 64 | L(+)-Ascorbic acid | 0.002 | 1.00 | 65 | 61 | 37 | 18 | 6 | 4 | 15 | 28 | 6870 | 6345 |
| Ex.9 | EDDS | 0.01 | 62 | Sodium sulfite | 0.20 | 57 | L(+)-Ascorbic acid | 0.002 | 1.00 | 52 | 64 | 42 | 16 | 6 | 7 | 24 | 32 | 7023 | 6539 |
| Ex.10 | EDDS | 0.02 | 62 | Sodium sulfite | 0.20 | 57 | L(+)-Ascorbic acid | 0.002 | 1.00 | 52 | 64 | 40 | 11 | 6 | 5 | 21 | 37 | 6211 | 7546 |
| Ex.11 | EDDS | 0.005 | 62 | Sodium sulfite | 0.20 | 57 | L(+)-Ascorbic acid | 0.002 | 1.00 | 52 | 62 | 46 | 10 | 7 | 7 | 26 | 35 | 6968 | 6799 |
| Ex.12 | EDDS | 0.01 | 62 | Sodium sulfite | 0.30 | 57 | L(+)-Ascorbic acid | 0.002 | 0.67 | 52 | 67 | 50 | 8 | 6 | 4 | 17 | 28 | 7579 | 6179 |
| Ex.13 | EDDS | 0.01 | 62 | Sodium sulfite | 0.10 | 57 | L(+)-Ascorbic acid | 0.002 | 2.0 | 52 | 65 | 45 | 11 | 6 | 8 | 21 | 32 | 7750 | 7276 |
| Ex.14 | EDDS | 0.01 | 62 | Sodium sulfite | 0.20 | 57 | L(+)-Ascorbic acid | 0.010 | 5.0 | 52 | 62 | 45 | 22 | 6 | 3 | 25 | 37 | 10570 | 8800 |
| Ex.15 | EDDS | 0.01 | 62 | Sodium sulfite | 0.20 | 57 | L(+)-Ascorbic acid | 0.001 | 0.50 | 52 | 66 | 46 | 10 | 7 | 8 | 23 | 34 | 7193 | 6629 |

\* Names of chelating agents: EDDS ... ethylenediamine-N,N'-disuccinic acid, DTPA ... diethylenetriaminepentaacetic acid, EDTMP ... ethylenediamine tetramethylenephosphonic acid

Reference Signs List

1: burette unit
3: clamp
4: measurement unit
5: conduit
10a: water-absorbent resin particles
11: stand
13: measurement table
13a: through hole
21: burette tube
22: cock
23: rubber stopper
24: cock
25: air inlet tube
31: cylinder

32: polyamide mesh
33: weight
50: saline
50a: support unit
51: column mount
52: column
53: stand
60: movable mount plate
61: measurement sample
70: drive unit
71: pulse motor
72: pulley
73: wire
80: measurement unit
81: load cell
82: precision spring
83: connecting shaft
84: disk-equipped pressure-sensitive shaft
90: weight

**Claims**

1. A method for producing water-absorbent resin particles comprising, in the following order:

   step 1 of polymerizing a water-soluble ethylenically unsaturated monomer to obtain hydrous gel particles;
   step 2 of adding a chelating agent, a sulfite-based compound, and an organic antioxidant to the hydrous gel particles; and
   step 3 of subjecting the hydrous gel particles to a surface-crosslinking treatment,
   wherein in step 2, a water content in the hydrous gel particles when adding the sulfite-based compound is 20% by mass or more and 75% by mass or less.

2. The method according to claim 1, wherein step 2 further includes the step of adjusting the water content in the hydrous gel particles.

3. The method according to claim 1 or 2, wherein in step 2, the water content in the hydrous gel particles when adding the chelating agent is 20% by mass or more.

4. The method according to claim 1 or 2, wherein in step 2, the water content in the hydrous gel particles when adding the organic antioxidant is 20% by mass or more.

5. The method according to claim 1 or 2, wherein in step 2, an amount of the sulfite-based compound to be added is 0.001 parts by mass or more and 3.0 parts by mass or less, per 100 parts by mass of the water-soluble ethylenically unsaturated monomer.

6. The method according to claim 1 or 2, wherein in step 2, an amount of the chelating agent to be added is 0.001 parts by mass or more and 2.0 parts by mass or less, per 100 parts by mass of the water-soluble ethylenically unsaturated monomer.

7. The method according to claim 1 or 2, wherein in step 2, an amount of the organic antioxidant to be added is 0.1 parts by mass or more and 20 parts by mass or less, per 100 parts by mass of the sulfite-based compound.

8. Water-absorbent resin particles,

   having a physiological saline absorption amount of 40 to 70 g/g,
   having a yellowness index of less than 40 after standing for 14 days in an environment of 70°C and 90% relative humidity, and
   having a gel strength of 5500 N/m$^2$ or more after standing for 14 hours in an environment of 37°C and 60% relative humidity.

9. An absorbent material comprising the water-absorbent resin particles according to claim 8.

10. An absorbent article comprising the absorbent material according to claim 9.

[Fig. 1]

[Fig. 2]

## INTERNATIONAL SEARCH REPORT

| International application No. |
|---|
| **PCT/JP2023/035291** |

| A. | CLASSIFICATION OF SUBJECT MATTER |
|---|---|

*C08F 2/10*(2006.01)i; *A61F 13/53*(2006.01)i; *C08F 8/00*(2006.01)i; *C08F 20/00*(2006.01)i; *C08K 3/30*(2006.01)i; *C08K 5/00*(2006.01)i; *C08L 101/14*(2006.01)i
FI: C08F2/10; C08F8/00; C08L101/14; C08K3/30; C08K5/00; A61F13/53 300; C08F20/00

According to International Patent Classification (IPC) or to both national classification and IPC

| B. | FIELDS SEARCHED |
|---|---|

Minimum documentation searched (classification system followed by classification symbols)

C08F2/10; 6/00-246/00; 301/00; A61F13/53; C08K3/30; C08K5/00; C08L101/14

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | WO 2021/006178 A1 (SUMITOMO SEIKA CHEMICALS CO., LTD.) 14 January 2021 (2021-01-14)<br>claims 1, 6-7, paragraphs [0004], [0005], [0034], [0035], [0038], [0041], [0042], [0074], [0075], [0113]-[0120], production example 1 | 1-10 |
| Y | JP 2005-029751 A (SUMITOMO SEIKA CHEMICALS CO., LTD.) 03 February 2005 (2005-02-03)<br>claims 1-2, 4-6, paragraphs [0013], [0016], [0018], [0023], [0060], [0062], tables 1, 2 | 1-10 |
| A | WO 2011/040530 A1 (NIPPON SHOKUBAI CO., LTD.) 07 April 2011 (2011-04-07)<br>entire text | 1-10 |
| A | JP 2018-131558 A (SDP GLOBAL CO., LTD.) 23 August 2018 (2018-08-23)<br>entire text | 1-10 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **22 November 2023** | **05 December 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

EP 4 600 273 A1

## INTERNATIONAL SEARCH REPORT
### Information on patent family members

International application No.

**PCT/JP2023/035291**

| Patent document cited in search report | | | Publication date (day/month/year) | Patent family member(s) | | | Publication date (day/month/year) |
|---|---|---|---|---|---|---|---|
| WO | 2021/006178 | A1 | 14 January 2021 | JP | 2021-11539 | A | |
| | | | | US | 2022/0314194 | A1 | |
| | | | | claims 1, 6-7, paragraphs [0004], [0005], [0038]-[0040], [0043], [0046]-[0048], [0081], [0082], [0124]-[0131], production example 1 | | | |
| | | | | JP | 2021-42286 | A | |
| | | | | CN | 114080426 | A | |
| | | | | KR | 10-2022-0024796 | A | |
| JP | 2005-029751 | A | 03 February 2005 | US | 2007/0111004 | A1 | |
| | | | | claims 1-2, 4-6, paragraphs [0011], [0015], [0017], [0039], [0049], tables 1, 2 | | | |
| | | | | WO | 2005/005549 | A1 | |
| | | | | EP | 1645596 | A1 | |
| | | | | CN | 1816596 | A | |
| | | | | TW | 200516106 | A | |
| WO | 2011/040530 | A1 | 07 April 2011 | CN | 102574100 | A | |
| | | | | KR | 10-2012-0081113 | A | |
| | | | | CN | 105363421 | A | |
| | | | | CN | 107051402 | A | |
| JP | 2018-131558 | A | 23 August 2018 | (Family: none) | | | |

Form PCT/ISA/210 (patent family annex) (January 2015)

28

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005029751 A